# EUROPÄISCHE PATENTSCHRIFT

(11) **EP 3 178 546 B1**
(45) Veröffentlichungstag und Bekanntmachung des Hinweises auf die Patenterteilung: **20.06.2018**
(21) Anmeldenummer: 16202445.9
(22) Anmeldetag: 06.12.2016
(51) Int. Cl.: B01F 11/00, B01F 15/02, B01F 13/06, B01F 13/00, B01F 15/00

(54) **MISCHVORRICHTUNG MIT BEDIENELEMENT UND DRUCKPUMPE ZUM MISCHEN VON POLYMETHYLMETHACRYLAT-KNOCHENZEMENT, UND VERFAHREN**
MIXING DEVICE WITH OPERATING ELEMENT AND PRESSURE PUMP FOR MIXING POLYMETHYLMETHACRYLATE BONE CEMENT, AND METHOD
DISPOSITIF DE MÉLANGE AVEC ORGANE DE COMMANDE ET POMPE DE REFOULEMENT POUR MÉLANGER DU CIMENT OSSEUX EN POLYMÉTHACRYLATE DE MÉTHYLE, ET PROCÉDÉ

(30) Priorität: 07.12.2015 DE 102015121276
(43) Veröffentlichungstag der Anmeldung: 14.06.2017
(73) Patentinhaber: Heraeus Medical GmbH, 61273 Wehrheim (DE)
(72) Erfinder: Kluge, Thomas, 56179 Vallendar (DE); Vogt, Sebastian, 99092 Erfurt (DE)
(74) Vertreter: Schultheiss & Sterzel Patentanwälte PartG mbB

(56) Entgegenhaltungen:
- EP-A1- 0 882 436
- WO-A1-2010/139078
- DE-U1- 20 005 333

## Beschreibung

Die Erfindung betrifft eine Mischvorrichtung zum Mischen von Polymethylmethacrylat-Knochenzement (PMMA-Knochenzement) aus zwei Ausgangskomponenten, insbesondere zum Mischen eines medizinischen Knochenzements, und zur Lagerung der Ausgangskomponenten. Die Erfindung betrifft ferner ein Verfahren zum Mischen von Polymethylmethacrylat-Knochenzement.

Gegenstand der Erfindung ist somit eine Mischvorrichtung für die Lagerung, Vermischung und gegebenenfalls auch den Austrag von Polymethylmethacrylat-Knochenzement. Weiterhin betrifft die Erfindung ein Verfahren zum Transfer von Monomerflüssigkeit in die Mischvorrichtung und ein Verfahren zum Vermischen der Komponenten von Polymethylmethacrlyat-Knochenzement.

Polymethylmethacrylat-Knochenzemente (PMMA-Knochenzemente) gehen auf die grundlegenden Arbeiten von Sir Charnley zurück. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere auf, die auf Basis von Methylmethacrylat und Comonomeren, wie Styren, Methylacrylat oder ähnlichen Monomeren durch Polymerisation, vorzugsweise Suspensionspolymerisation, hergestellt sind. Zudem weist das Knochenzementpulver einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Zementteigs, bis dieser erstarrt.

Das am häufigsten in Polymethylmethacrylat-Knochenzementen verwendete Monomer ist Methylmethacrylat. Redoxinitiatorsysteme bestehen üblicherweise aus Peroxiden, Beschleunigern sowie gegebenenfalls geeigneten Reduktionsmitteln. Eine Radikalbildung erfolgt nur dann, wenn alle Bestandteile der Redoxinitiatorsysteme zusammenwirken. Deshalb werden die Bestandteile des Redoxinitiatorsystems in den separaten Ausgangskomponenten so angeordnet, dass diese keine radikalische Polymerisation auslösen können. Die Ausgangskomponenten sind bei geeigneter Zusammensetzung lagerstabil. Erst bei Vermischung der beiden Ausgangskomponenten zu einem Zementteig reagieren die zuvor getrennt in den beiden Pasten, Flüssigkeiten oder Pulvern gelagerten Bestandteile des Redoxinitiatorsystems, wobei Radikale gebildet werden, welche die radikalische Polymerisation des wenigstens einen Monomers auslösen. Die radikalische Polymerisation führt dann unter Verbrauch des Monomers zu Bildung von Polymeren, wobei der Zementteig aushärtet.

PMMA-Knochenzemente können in geeigneten Mischbechern mit Hilfe von Spateln durch Vermischen des Zementpulvers mit der Monomerflüssigkeit vermischt werden. Nachteilig ist an dieser Vorgehensweise, dass Lufteinschlüsse im gebildeten Zementteig vorhanden sein können, die später eine mechanische Destabilisierung des Knochenzements verursachen können.

Zur Vermeidung von Lufteinschlüssen im Knochenzementteig wurden eine Vielzahl von Vakuum-Zementiersystemen offen gelegt, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, WO 00/35506 A1, EP 1 005 901 A2, US 5 344 232 A. Bei den dargelegten Vakuum-Zementiersystemen ist es erforderlich, zur Erzeugung des Unterdrucks eine externe Vakuumpumpe anzuschließen. Diese werden im Allgemeinen mit Druckluft unter Nutzung des Venturi-Prinzips betrieben. Die für den Betrieb der Vakuumpumpen notwendige Druckluft wird entweder aus stationären Druckluftanlagen oder auch aus elektrisch betriebenen Kompressoren bezogen. Daneben ist es auch möglich, für die Vakuumerzeugung elektrisch betriebene Vakuumpumpen zu verwenden.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche geschlossenen Full-Prepacked-Mischsysteme wurden mit der EP 0 692 229 A1, der DE 10 2009 031 178 B3, der US 5 997 544 A, der US 6 709 149 B1, der DE 698 12 726 T2 und der US 5 588 745 A vorgeschlagen. Das Patent DE 10 2009 031 178 B3 offenbart eine Vakuum-Mischvorrichtung mit einem zweiteiligen Austragskolben, der auch für eine erfindungsgemäße Mischvorrichtung einsetzbar ist. Dabei wird eine Kombination aus einem Gasdurchlässigen Sterilisationskolben und einem Gas-undurchlässigen Dichtungskolben verwendet.

In der WO 00/35506 A1 wird eine Vorrichtung vorgeschlagen, bei der PMMA-Knochenzementpulver in einer Kartusche gelagert wird, wobei das Zementpulver das gesamte Volumen der Kartusche ausfüllt und die Zwischenräume zwischen den Partikeln des Zementpulvers ein solches Volumen hat, das dem Volumen der Monomerflüssigkeit entspricht, das zur Herstellung von Knochenzementteig mit dem in der Kartusche gelagerten Zementpulver notwendig ist. Diese Vorrichtung ist so aufgebaut, dass durch Vakuumeinwirkung die Monomerflüssigkeit von oben in die Kartusche eingeleitet wird, wobei hierzu ein Vakuum an einem Vakuumanschluss an der Unterseite der Kartusche angelegt wird. Dadurch wird die Monomerflüssigkeit durch das Zementpulver gezogen, wobei die in den Zwischenräumen der Zementpartikel befindliche Luft durch die Monomerflüssigkeit verdrängt wird. Auf eine mechanische Durchmischung des gebildeten Zementteigs mit einem Rührer wird dabei verzichtet.

Nachteilig an diesem System ist, dass Zementpulver, die schnell mit der Monomerflüssigkeit anquellen, mit dieser Vorrichtung nicht angemischt werden können, weil die schnell anquellenden Zementpulverpartikel nach einem Eindringen der Monomerflüssigkeit in das Zementpulver von ungefähr 1 bis 2 cm eine gelartige Barriere bilden und die Migration der Monomerflüssigkeit durch das gesamte Zementpulver behindern. Weiterhin kann bei Vakuumeinwirkung nicht ausgeschlossen werden, dass nach vollständiger Durchdringung des Zementpulvers durch die Monomerflüssigkeit die Monomerflüssigkeit über den Vakuumanschluss abgesaugt wird. Dann steht nicht genügend Monomerflüssigkeit für die Aushärtung durch radikalische Polymerisation zur Verfügung beziehungsweise das Mischungsverhältnis wird ungewollt verändert und damit auch die Konsistenz des Knochenzements. Weiterhin ist es problematisch, dass die zwischen den Zementpulverpartikeln eingeschlossen Luft durch die Monomerflüssigkeit von oben nach unten verdrängt werden soll, weil die gegenüber der Monomerflüssigkeit spezifisch leichtere Luft auf Grund der Schwerkraft das Bestreben hat, im Zementpulver nach oben zu wandern und nicht nach unten in Richtung Vakuumanschluss zu migrieren. Auch die DE 200 05 333 U1 offenbart eine Vakuummischvorrichtung und ein Verfahren zur Vermischung von PMMA-Knochenzement. Bei der Verwendung von Mischvorrichtungen zur Zementierung müssen externe Vakuumpumpen beigestellt werden. Diese Vakuumpumpen sind kostenintensiv und müssen nach der Anwendung gereinigt werden. Weiterhin sind Vakuumschläuche zur Verbindung der Vakuumpumpen mit den Mischvorrichtungen notwendig. Diese Vakuumschläuche müssen den Mischvorrichtungen beigelegt sein. Vor dem Mischen mit einer Mischvorrichtung muss daher zuerst die Vakuumpumpe im Operations-Saal (OP-Saal) aufgebaut und an eine Energiequelle, wie beispielsweise Druckluft, oder an eine elektrische Spannungsquelle angeschlossen werden. Danach wird die Vakuumpumpe mit einem Vakuumschlauch mit der Mischvorrichtung verbunden. Diese Montageschritte kosten wertvolle OP-Zeit und sind potentiell fehlerbehaftet. Die Vakuumpumpe und die Verbindungsleitungen zur Mischvorrichtung und zu externen Energiequellen und Versorgungsleitungen benötigen Platz und stellen potentielle Stolperfallen und Hindernisse dar, die den gelegentlich hektischen Ablauf während einer Operation stören können.

Ein weiteres interessantes Konzept wird mit der EP 1 886 647 A1 vorgeschlagen. Das Zementpulver ist dabei in einer evakuierten Kartusche gelagert und die Monomerflüssigkeit befindet sich in einem separaten Behälter. Beim Öffnen der unter Unterdruck stehenden Kartusche wird die Monomerflüssigkeit in die Kartusche gesaugt ohne dass Luft einströmt. Es entsteht ein von Lufteinschlüssen freier Knochenzementteig. Dieses Konzept erfordert es, dass die Kartusche während der Lagerung vor ihrer Verwendung vakuumdicht verschlossen bleibt und keine unsterile Luft eindringen kann. Die Kartusche muss dazu stabil hermetisch abgedichtet sein. Nachteilig ist hieran also, dass der Aufbau aufwendig ist und dass der Inhalt der Kartusche nach dem Einsaugen der Monomerflüssigkeit nicht von einem extern zu bedienenden Mischsystem durchmischt werden kann, da eine Durchführung für einen Mischstab oder ein Mischrohr nicht ohne weiteres dauerhaft vakuumdicht ist. Bei bekannten Full-Prepacked-Mischsysteme wird meist Vakuum oder ein Unterdruck benutzt, um die Monomerflüssigkeit in das Zementpulver zu überführen.

Die Aufgabe der Erfindung besteht also darin, die Nachteile des Stands der Technik zu überwinden. Insbesondere sollen die Nachteile der bekannten Vakuummischvorrichtungen als Full-Prepacked-Systeme mit externer Vakuumquelle überwunden werden, ohne dass ein Unterdruck über lange Zeit gehalten werden muss. Die Erfindung hat dabei unter anderem die Aufgabe, eine einfache, geschlossene Vorrichtung zu entwickeln, bei der Polymethylmethacrylat-Knochenzementpulver (Zementpulver) und Monomerflüssigkeit in separaten Kompartimenten gelagert und anschließend vermischt werden können. Vom medizinischen Anwender soll das Polymethylmethacrylat-Knochenzementpulver mit der Monomerflüssigkeit innerhalb der Vorrichtung zusammengeführt und vermischt werden können, ohne dass beide Komponenten mit dem medizinischen Anwender in Berührung kommen. Ein Kontakt des medizinischen Anwenders mit dem Polymethylmethacrylat-Knochenzementpulver und mit der Monomerflüssigkeit soll möglichst ausgeschlossen sein. Bei der zu entwickelnden Vorrichtung handelt es sich um ein Full-Prepacked-Mischsystem. Die Vorrichtung soll so beschaffen sein, dass ein Transfer der Monomerflüssigkeit in das Polymethylmethacrylat-Knochenzementpulver ohne die Verwendung von durch Druckluft oder Kompressoren angetriebenen externen Pumpen, transferiert werden kann. Wichtig ist ferner, dass die Vorrichtung ohne externe Energiequellen, wie Druckluft, Vakuum oder elektrischer Strom, auch unter einfachsten äußeren Bedingungen funktionsfähig und zuverlässig die Herstellung von Knochenzementteig gewährleistet. Die Vorrichtung soll ohne zusätzliche technische Ausrüstung autonom verwendungsfähig sein.

Die Mischvorrichtung soll maximal vereinfacht sein und in der Lage sein, einen Transfer von Monomerflüssigkeit aus einem Monomerbehälter in eine mit Zementpulver gefüllte Kartusche zu ermöglichen. Es soll dann ferner ein Verfahren bereitgestellt werden, das einen Monomertransfer und ein Mischen der Komponenten in Full-Prepacked-Mischvorrichtungen ermöglicht. Weiterhin soll die zu entwickelnde Mischvorrichtung hauptsächlich aus kostengünstigem Kunststoff gefertigt werden können.

Die Erfindung hat ferner die Aufgabe, eine einfache geschlossene Prepack-Mischvorrichtung zu entwickeln, bei der Polymethylmethacrylat-Knochenzementpulver und Monomerflüssigkeit in separaten Kompartimenten gelagert und anschließend vermischt werden können. Unmittelbar vor der Vermischung der Komponenten soll die Monomerflüssigkeit ohne Nutzung von externen Vakuumquellen, externen elektrischen Antrieben und externen Druckluftantieben in das Zementpulver transferiert werden. Mit der Prepack-Mischvorrichtung soll unabhängig von zusätzlichen externen Vorrichtungen rein durch manuelle Betätigung Polymethylmethacrylat-Knochenzement erzeugt werden können. Dabei soll die manuelle Betätigung so weit wie möglich vereinfacht werden. Vorzugsweise soll das Öffnen der Monomerampulle, beziehungsweise der Monomerampullen, der Monomertransfer und die Vermischung der Zementkomponenten nur durch möglichst eine einfache Bewegung eines Bedienelements bewirkt werden, die besonders bevorzugt nur wenige Male, beispielsweise 3 bis 5 mal, wiederholt werden muss. Dadurch soll die Benutzung der Mischvorrichtung für den Anwender maximal vereinfacht werden, so dass kostenintensive Schulungen eingeschränkt beziehungsweise eingespart werden können. Weiterhin soll durch eine maximal vereinfachte Bedienung der Mischvorrichtung mögliche Bedienungsfehler minimiert werden, wodurch die Patientensicherheit erhöht wird.

Vom medizinischen Anwender soll das Polymethylmethacrylat-Knochenzementpulver mit der Monomerflüssigkeit innerhalb der Mischvorrichtung zusammengeführt und vermischt werden können, ohne dass beide Zementkomponenten mit dem medizinischen Anwender in Berührung kommen. Ein Kontakt des medizinischen Anwenders mit dem Polymethylmethacrylat-Knochenzementpulver und mit der Monomerflüssigkeit muss ausgeschlossen sein. Bei der zu entwickelnden Mischvorrichtung handelt es sich um eine Full-Prepack Mischvorrichtung. Die Mischvorrichtung soll so beschaffen sein, dass ein Transfer der Monomerflüssigkeit in das Polymethylmethacrylat-Knochenzementpulver ohne die Verwendung von externen Vakuumpumpen erfolgt. Des Weiteren soll die Mischvorrichtung ohne externe Energiequellen, wie Druckluft, Vakuum oder elektrischer Strom, auch unter einfachsten äußeren Bedingungen funktionsfähig und zuverlässig die Herstellung von Knochenzementteig gewährleisten. Besonders bevorzugt soll die Mischvorrichtung möglichst weitgehend auch ohne interne Energiespeicher, wie beispielsweise Batterien oder auch mechanische Energiespeicher auskommen Die Mischvorrichtung soll ohne zusätzliche technische Ausrüstung autonom verwendungsfähig sein.

Des Weiteren soll eine kostengünstig zu fertigende und zuverlässig funktionierende Mischvorrichtung zum Mischen eines medizinischen Zements und gegebenenfalls zur Lagerung der Ausgangskomponenten sowie ein Verfahren zum Mischen des Knochenzements gefunden werden, bei dem eine möglichst einfache manuelle Bedienung zum Mischen der Ausgangskomponenten angewendet werden kann.

Die Hauptkomponente des Polymethylmethacrylat-Knochenzements als Mischgut soll ein Pulver sein und die zweite Komponente soll in Form einer Flüssigkeit vorliegen. Die beiden Ausgangskomponenten des Knochenzements sollen bevorzugt in der Mischvorrichtung getrennt gelagert werden können und durch Anwendung der Vorrichtung sicher zusammengeführt werden können.

Die Aufgaben der Erfindung werden gelöst durch eine Mischvorrichtung zum Mischen von Polymethylmethacrylat-Knochenzement aus einer Monomerflüssigkeit und einem Zementpulver, die Mischvorrichtung aufweisend
zumindest eine Kartusche umfassend einen evakuierbaren Innenraum zum Mischen des Knochenzements,
eine Mischeinrichtung zum Durchmischen des Inhalts des Innenraums der zumindest einen Kartusche, die beweglich im Innenraum angeordnet ist,
eine Aufnahme zur Aufnahme eines die Monomerflüssigkeit enthaltenden separaten Behälters oder aufweisend einen integrierten Behälter enthaltend die Monomerflüssigkeit,
eine Öffnungseinrichtung, die im Bereich der Aufnahme gegen die Aufnahme beweglich angeordnet ist, so dass durch eine Bewegung der Öffnungseinrichtung ein in der Aufnahme angeordneter separater Behälter mit der Öffnungseinrichtung zu öffnen ist, oder die Öffnungseinrichtung im Bereich des integrierten Behälters gegen den integrierten Behälter beweglich angeordnet ist, so dass durch eine Bewegung der Öffnungseinrichtung der integrierte Behälter mit der Öffnungseinrichtung zu öffnen ist,
eine Druckpumpe, in der ein beweglicher Kolben zum Fördern einer Flüssigkeit angeordnet ist und der einen Pumpraum der Druckpumpe begrenzt, und
eine Verbindungsleitung, die den Innenraum der zumindest einen Kartusche mit dem Pumpraum der Druckpumpe verbindet, wobei
die Mischvorrichtung ein von außen bedienbares Bedienelement aufweist, wobei mit dem Bedienelement der Kolben in der Druckpumpe manuell bewegbar ist, und wobei mit demselben Bedienelement die Öffnungseinrichtung gegen die Aufnahme oder gegen den integrierten Behälter zu bewegen ist, und
mit demselben Bedienelement die Mischeinrichtung im Innenraum der Kartusche zum Durchmischen des Inhalts des Innenraums der Kartusche bewegbar ist.

Bevorzugt ist die Mischvorrichtung auch zum Lagern der Ausgangskomponenten des Polymethylmethacrylat-Knochenzements geeignet. Besonders bevorzugt sind die Monomerflüssigkeit und/oder das Zementpulver in der Mischvorrichtung enthalten. Bei den Ausgangskomponenten des Polymethylmethacrylat-Knochenzements handelt es sich um das Zementpulver und die Monomerflüssigkeit, wobei die Monomerflüssigkeit bevorzugt in einer Glasampulle enthalten ist, die als separater Behälter in der Aufnahme angeordnet ist. Die Monomerflüssigkeit kann aber auch in einem Folienbeutel als separater Behälter enthalten sein oder sie kann in dem integrierten Behälter enthalten sein, der durch die Aufnahme beziehungsweise die Mischvorrichtung selbst gebildet wird.

Der Begriff "Mischvorrichtung" soll dahingehend verstanden werden, dass die Ausgangskomponenten des Knochenzements, also die Monomerflüssigkeit und das Zementpulver, innerhalb der Vorrichtung gemischt werden können.

Aufgrund der speziellen Anforderungen, wie die des geringen Volumens des Innenraums der Kartusche, sind aufwendigere Druckpumpen oder Transfersysteme nicht notwendig.

Bevorzugt kann vorgesehen sein, dass die Druckpumpe in die Mischvorrichtung integriert ist.

Bevorzugt kann erfindungsgemäß auch vorgesehen sein, dass das Zementpulver in dem Innenraum der Kartusche enthalten ist. Das Zementpulver muss dann nicht in den Innenraum der Kartusche eingefüllt werden.

Durch die Kopplung der Öffnungseinrichtung mit dem Bedienelement ist der Behälter in der Aufnahme oder ist der integrierte Behälter durch manuelles Bedienen des Bedienelements mit der Öffnungseinrichtung zu öffnen.

Die Mischvorrichtung umfasst entweder eine Aufnahme, in die ein separater Behälter, wie beispielsweise eine Glasampulle oder ein Folienbeutel, die oder der die Monomerflüssigkeit enthält, eingesetzt werden kann, oder einen integrierten Behälter, der als integraler Teil der Mischvorrichtung ausgebildet ist und in dem die Monomerflüssigkeit bereits enthalten ist.

Es kann auch vorgesehen sein, dass mehr als eine Kartusche mit jeweils einem Innenraum vorgesehen ist, wobei dann in jedem Innenraum eine Mischeinrichtung vorgesehen ist und jeder Innenraum über eine Verbindungsleitung mit dem Pumpraum der Druckpumpe oder mit jeweils einem Pumpraum einer Mehrzahl separater Druckpumpen verbunden ist.

Bevorzugt ist die Aufnahme auch zur Fixierung einer Glasampulle beziehungsweise der Glasampulle in der Aufnahme geeignet und vorgesehen. Die Glasampulle muss hierzu selbstverständlich passend geformt sein. Beispielsweise kann die Glasampulle in Presspassung in die Aufnahme eingesteckt werden.

Es kann erfindungsgemäß vorgesehen sein, dass die Aufnahme auf einer Seite mit einem Deckel verschlossen ist. Dabei kann bevorzugt in dem Deckel zumindest eine gasdurchlässige Öffnung vorgesehen sein, durch die Gas in die Aufnahme einströmen beziehungsweise nachströmen kann, wenn die Monomerflüssigkeit aus der Aufnahme heraus fließt. Hierdurch soll vermieden werden, dass sich in der Aufnahme ein Unterdruck bildet, der dem Fluss der Monomerflüssigkeit in den Pumpraum entgegenwirkt.

Bevorzugt weist die Kartusche eine fluiddichte oder druckdichte Durchführung auf, durch die ein Stab, eine Kabel oder eine Mischwelle durchgeführt ist, mit dem die Mischeinrichtung von außerhalb der Kartusche zu bewegen ist. Dazu ist der Stab, das Kabel oder die Mischwelle bevorzugt drehbar und in Längsrichtung verschiebbar in der Durchführung gelagert. Mit der Mischeinrichtung kann der Inhalt der Kartusche gut durchmischt werden.

Bevorzugte Ausführungsformen können sich dadurch auszeichnen, dass die Mischvorrichtung weniger als 10 kg Gesamtgewicht hat, besonders bevorzugt weniger als 2 kg Gesamtgewicht hat, besonders bevorzugt weniger als 1 kg.

Diese geringen Gewichte sind mit dem erfindungsgemäßen Aufbau der Mischvorrichtung mit manuellem bedienbarem Bedienelement und der Druckpumpe möglich. Das geringe Gewicht hat den Vorteil, dass die Mischvorrichtung mitgenommen werden kann, transportabel ist und ohne Anschluss an Versorgungsleitungen und ohne große Vorbereitungen einsetzbar ist.

Es kann erfindungsgemäß vorgesehen sein, dass ein Sieb und/oder ein Filter das, der oder die unterhalb der Aufnahme, des separaten Behälters oder unterhalb des integrierten Behälters angeordnet ist oder sind, so dass der Inhalt des geöffneten integrierten Behälters oder separaten Behälters durch das Sieb und/oder den Filter fließt.

Damit können Glassplitter, Folienschnipsel oder andere Rückstände des Verschlusses beziehungsweise des separaten oder integrierten Behälters, die beim Öffnen des separaten oder integrierten Behälters mit der Öffnungseinrichtung entstanden sind, zurückgehalten werden. Damit wird ein Verstopfen der Verbindungsleitung zum Innenraum der Kartusche sowie eine Verunreinigung des herzustellenden Knochenzements verhindert.

Erfindungsgemäße Mischvorrichtungen zeichnen sich dadurch aus, dass sie ohne einen elektrischen Antrieb auskommen. Es kann also erfindungsgemäß vorgesehen sein, dass die Mischvorrichtung keinen elektrischen Antrieb aufweist oder zumindest die Druckpumpe, die Öffnungseinrichtung und die Mischeinrichtung nicht mit einem elektrischen Antrieb angetrieben werden. Stattdessen erfolgt der Antrieb dieser Bauteile erfindungsgemäß über das manuell bedienbare Bedienelement. Ebenso können erfindungsgemäße Mischvorrichtungen ohne Elektronik beziehungsweise elektronische Bauteile aufgebaut werden. Eine erfindungsgemäße Mischvorrichtung kann sich also auch dadurch auszeichnen, dass darin keine Elektronik verbaut ist oder dass zumindest zum Antrieb der Druckpumpe, der Öffnungseinrichtung und der Mischeinrichtung keine Elektronik oder keine elektronischen Bauteile angewendet werden. Elektromotoren oder Kompressoren werden also nicht zum Aufbau erfindungsgemäßer Mischvorrichtungen benötigt.

Des Weiteren kann erfindungsgemäß vorgesehen sein, dass die Mischvorrichtung keinen Energiespeicher aufweist, insbesondere keinen elektrischen Energiespeicher, wie beispielsweise eine Batterie oder einen Akkumulator, und keinen Druckgasspeicher, wie beispielsweise eine CO₂-Druckgaspatrone, oder dass zumindest kein Energiespeicher, vorzugsweise kein elektrischer Energiespeicher oder Druckgasspeicher, zum Antrieb der Druckpumpe, der Öffnungseinrichtung und der Mischeinrichtung verwendet werden. Bevorzugt weist die Mischvorrichtung aber auch keine elastischen Energiespeicher, wie beispielsweise gespannte Federn, auf.

Bei erfindungsgemäßen Mischvorrichtungen kann vorgesehen sein, dass das Bedienelement mit dem Kolben derart verbunden oder verbindbar ist, dass der Kolben in der Druckpumpe durch Bedienen des Bedienelements manuell bewegbar ist, vorzugsweise in zumindest einer Richtung manuell bewegbar ist, besonders bevorzugt in einer Richtung manuell bewegbar ist.

Hiermit wird erreicht, dass der Kolben, insbesondere unmittelbar, mit dem Bedienelement bewegt werden kann. Dabei kann vorgesehen sein, dass sich der Kolben erst nach einem ersten Bedienen des Bedienelements mit dem Bedienelement in der Art verbindet, dass der Kolben durch eine weitere Bedienung des Bedienelements in der Druckpumpe bewegt wird. Für die Ausführung des Bedienelements ist dabei ein Hebel besonders gut geeignet, der um eine Achse hin und her gezogen beziehungsweise gedrückt beziehungsweise geschwenkt werden kann, so dass nach einem ersten Bewegen des Hebels der separate Behälter in der Aufnahme geöffnet wird oder der integrierte Behälter geöffnet wird und sich der Kolben und/oder die Mischeinrichtung dabei mit dem Bedienelement derart verbindet oder verbinden, dass bei einer umgekehrten Bewegung des Hebels der Kolben in der Druckpumpe und/oder die Mischeinrichtung in dem Innenraum der Kartusche bewegt wird oder werden. Zudem sind mit einem Hebel problemlos große Kräfte manuell in die Mischvorrichtung zu übertragen.

Des Weiteren kann vorgesehen sein, dass die Aufnahme zumindest bereichsweise geschlossene Seitenwände zur Aufnahme einer Glasampulle als separaten Behälter aufweist, wobei die Aufnahme zumindest eine verformbare geschlossene Seitenwand aufweist und gegenüber der verformbaren Seitenwand ein Stützelement vorgesehen ist, wobei die Öffnungseinrichtung über das Bedienelement gegen die verformbare Seitenwand der Aufnahme zu pressen ist, so dass sich die verformbare Seitenwand derart verformt, dass eine in der Aufnahme angeordnete passende Glasampulle mit der Öffnungseinrichtung aufzubrechen ist.

Durch diese Maßnahme kann die Aufnahme weitgehend nach außen geschlossen werden. Zudem kann hierdurch sichergestellt werden, dass eine Glasampulle auch innerhalb der geschlossenen Aufnahme geöffnet werden kann, ohne dass ohne weiteres Monomerflüssigkeit aus der Mischvorrichtung austreten kann, wodurch die Gefahr einer Kontamination der Umgebung der Mischvorrichtung mit dem Inhalt der Kartusche, insbesondere mit der Monomerflüssigkeit, ausgeschlossen werden kann oder die Gefahr hierfür zumindest stark reduziert wird.

Mit einer Weiterbildung der vorliegenden Erfindung wird vorgeschlagen, dass die Öffnungseinrichtung einen ersten Hebel aufweist, der um eine erste Achse drehbar gegen die Aufnahme oder den integrierten Behälter gelagert ist, wobei ein freies Ende des ersten Hebels gegen eine verformbare Seitenwand der Aufnahme oder den integrierten Behälter drückbar ist, wobei das Bedienelement durch einen zweiten Hebel gebildet ist, der um eine zweite Achse drehbar gegen die Aufnahme oder den integrierten Behälter gelagert ist, wobei die zweite Achse den zweiten Hebel in einen kurzen Hebelarm und einen langen Hebelarm teilt, wobei ein Ende des kurzen Hebelarms durch manuelles Bedienen des langen Hebelarms gegen den ersten Hebel zu drücken ist, so dass das freie Ende des ersten Hebels gegen die verformbare Seitenwand drückt und diese derart verformt, dass eine in der Aufnahme befindlicher separater Behälter zu öffnen ist, oder das freie Ende des ersten Hebels gegen den integrierten Behälter drückt, so dass der integrierte Behälter sich zu einer Verbindung in den Pumpraum öffnet.

Dabei kann vorgesehen sein, dass der separate Behälter eine zur Aufnahme passende Glasampulle ist, die durch den Druck des freien Endes des ersten Hebels aufzubrechen ist oder ein in der Aufnahme angeordneter Folienbeutel ist, der durch den Druck des freien Endes des ersten Hebels aufzustechen, aufzuschlitzen oder aufzureißen ist.

Ferner kann bei solchen Ausführungen vorgesehen sein, dass am freien Ende des ersten Hebels auf der zur Aufnahme zugewandten Seite eine Kante angeordnet ist. Das Längenverhältnis des langen Hebelarms zum kurzen Hebelarm beträgt bevorzugt mindestens 5 zu 1. Des Weiteren kann vorgesehen sein, dass der zweite Hebel in der gleichen Ebene zu drehen ist, wie der erste Hebel, wobei die Bewegung des zweiten Hebels in die Bewegung des ersten Hebels eingreift. Bevorzugt kann auch vorgesehen sein, dass die zweite Achse des zweiten Hebels oberhalb der ersten Achse des ersten Hebels angeordnet ist, wobei vorzugsweise die erste Achse des ersten Hebels und die zweite Achse des zweiten Hebels parallel zueinander angeordnet sind.

Mit derartigen Mischvorrichtungen mit Glasampulle gelingt es, eine Glasampulle großflächig innerhalb der Vorrichtung beziehungsweise der Zementiervorrichtung aufzubrechen, so dass die Monomerflüssigkeit aus der Glasampulle in kurzer Zeit ausströmt, in den Pumpraum der Druckpumpe fließt und dadurch zum Mischen mit dem medizinischen Knochenzementpulver bereitsteht. Mit Hilfe der beiden Hebel, die in Wechselwirkung miteinander stehen, ist es möglich, den Druck auf die Glasampulle in Richtung des Sitzes der Glasampulle in der Aufnahme zu richten, so dass ein Ausweichen der Glasampulle aus der Aufnahme heraus ausgeschlossen werden kann. Gleichzeitig kann ein sehr genau definierter lokaler Druck auf die Glasampulle ausgeübt werden, mit dem die Glasampulle in der Mischvorrichtung aufgebrochen werden kann. Mit Hilfe der verformbaren Seitenwand kann dafür gesorgt werden, dass die Kraft durch diese Seitenwand ins Innere der Aufnahme auf die Glasampulle übertragen wird, wobei die Aufnahme dabei geschlossen bleibt. Ein Austreten der Monomerflüssigkeit aus der Aufnahme kann somit ausgeschlossen werden. Mit Hilfe des Siebs und/oder des Filters können Glassplitter, die beim Öffnen der Glasampulle entstehen können, zurückgehalten werden. Die Monomerflüssigkeit ist dann zum Mischen mit dem Knochenzementpulver nutzbar.

Der besondere Vorteil der erfindungsgemäßen Vorrichtung besteht auch darin, dass beliebige Glasampullen, unabhängig von der Ampullenlänge und der Geometrie des Ampullenkopfs, sicher geöffnet werden können, wenn der Ampullendurchmesser gleich oder etwas größer ist als der Innendurchmesser des Ampullenhalters beziehungsweise der Aufnahme. Weiterhin ist es ein besonderer Vorteil, dass beim Brechen der Ampullenwand im Bereich des Ampullenbodens die in der Glasampulle enthaltene Flüssigkeit unabhängig von der Oberflächenspannung sofort komplett ausfließt. Dagegen fließt bei konventionellen Ampullenbrechern die Flüssigkeit nach Abtrennen des Ampullenkopfs durch den relativ engen Querschnitt des Ampullenhalses deutlich langsamer aus. Dabei werden halbwegs große Auslaufgeschwindigkeiten nur erzielt, wenn entweder die Glasampulle im Winkel von ca. 45° mit dem Ampullenhals nach unten gehalten wird oder wenn der Querschnitt des Ampullenhalses so groß ist, dass die Oberflächenspannung der Flüssigkeit den Meniskus der Flüssigkeit nicht im Ampullenhals halten kann.

Bevorzugt ist die Aufnahme ein Hohlzylinder. Ebenfalls bevorzugt besteht die Aufnahme aus einem Elastomer oder umfasst einen Einsatz aus einem Elastomer, wie beispielsweise ein Ethylen-Propylen-Dien-Kautschuk (EPDM).

Ferner kann dabei vorgesehen sein, dass in der Aufnahme ein Absatz zur Auflage der Glasampulle angeordnet ist, wobei der Absatz kleiner als die Hälfte der Fläche des Ampullenbodens oder des Ampullenquerschnitts ist. Dabei kann wiederum bevorzugt vorgesehen sein, dass der Absatz derart in der Aufnahme angeordnet ist, dass der Abstand zwischen dem Absatz und einem darunter angeordneten Sieb und/oder Filter gleich oder größer ist als der Außendurchmesser der einzusetzenden Glasampulle.

Mit einer besonders einfach und kostengünstig zu realisierenden Mischvorrichtung wird vorgeschlagen, dass das Bedienelement manuell beweglich ist, vorzugsweise ein um eine Achse schwenkbarer Hebel ist, wobei das Bedienelement derart mit der Öffnungseinrichtung, der Druckpumpe und der Mischeinrichtung in Wirkverbindung steht oder in Wirkverbindung zu bringen ist, dass bei einem ersten Bedienen des Bedienelements ein separater Behälter in der Aufnahme oder der integrierte Behälter zu öffnen ist und bei einem weiteren Bedienen des Bedienelements der Kolben in der Druckpumpe anzutreiben ist und die Mischeinrichtung im Innenraum anzutreiben ist.

Hiermit kann eine manuell aufzubringende Kraft, die auf das Bedienelement, insbesondere den Hebel, einwirkt, dazu verwendet werden, zunächst den separaten oder integrierten Behälter zu öffnen und anschließend die Druckpumpe und die Mischeinrichtung mit demselben Bedienelement anzutreiben beziehungsweise zu bewegen. Hebel als Bedienelemente sind für die Übertragung manueller Kraft in die Mischvorrichtung besonders gut geeignet. Auch weil über die Länge des Hebelarms eine Verstärkung der nutzbaren Kraft möglich ist.

Dabei kann vorgesehen sein, dass der Kolben der Druckpumpe und/oder die Mischeinrichtung über ein flexibles Kabel und/oder eine Stange anzutreiben sind, wobei an dem flexiblen Kabel und/oder der Stange ein Rastmittel vorgesehen ist, das nach erstmaligem Bedienen des Bedienelements in ein Gegenrastmittel am Bedienelement oder in ein mit dem Bedienelement verbundenes Gegenrastmittel greift, so dass bei einem der Rastung nachfolgenden Bedienen des Bedienelements der Kolben der Druckpumpe und/oder die Mischeinrichtung über das Kabel und/oder die Stange mit dem Bedienelement anzutreiben sind.

Hiermit kann eine Übertragung der Kraft von dem Bedienelement auf den Kolben und/oder die Mischeinrichtung erfolgen. Die flexiblen Kabel sind besonders gut zur Übertragung der Kraft geeignet, da sich damit ohne aufwendige Mechanik die Richtung der Kraft umlenken lässt. Vorzugsweise ist das flexible Kabel ausreichend steif beziehungsweise starr, so dass sich die Mischeinrichtung in beide Richtungen hin und her bewegen lässt. Hierzu kann das Kabel geführt und oder gestützt werden. Beispielsweise kann das Kabel hierzu in einem Kanal geführt sein oder es kann an geeigneten Stellen durch ein Gehäuse und durch Streben und/oder Umlenkrollen im Gehäuse gestützt beziehungsweise umgelenkt werden. Durch die Anwendung des Rastmittels und des Gegenrastmittels kann sichergestellt werden, dass zunächst der separate Behälter in der Aufnahme oder der integrierte Behälter geöffnet wird und die Monomerflüssigkeit vollständig oder weitgehend in den Pumpraum fließt, bevor die Druckpumpe beziehungsweise der Kolben der Druckpumpe und/oder die Mischeinrichtung bedient wird. Damit kann beispielsweise verhindert werden, dass die Druckpumpe schon betrieben wird, bevor die Monomerflüssigkeit im Pumpraum zur Verfügung steht.

Es wird ferner vorgeschlagen, dass die Mischeinrichtung durch Bedienen des Bedienelements in dem Innenraum axial in Längsrichtung beweglich ist.

Hiermit wird insbesondere für einen zylindrischen Innenraum eine sehr weitreichende Durchmischung des Innenraums erreicht. Gleichzeitig kann die Mischeinrichtung kompakt aufgebaut werden, da sie sich nicht entlang der gesamten Länge des Innenraums erstrecken muss.

Bevorzugt kann auch vorgesehen sein, dass die Mischeinrichtung durch Bedienen des Bedienelements in dem Innenraum um die Längsachse des Innenraums drehbar ist, wobei hierzu vorzugsweise sich ein mit der Mischeinrichtung verbundener Zylinder mit einem Außengewinde in einer feststehenden Hülse mit einem passenden Innengewinde bewegt, so dass bei einer Bewegung des Zylinders entlang der Längsrichtung innerhalb der Hülse eine Drehung des Zylinders erzwungen wird, wobei sich die Drehung des Zylinders auf die Mischeinrichtung im Innenraum der Kartusche überträgt.

Hierdurch wird eine noch stärkere Durchmischung des Inhalts des Innenraums erreicht. Auch an der Wand des Innenraums anliegender Knochenzement kann dadurch effektiv durchmischt werden. Der Knochenzement wird so schneller und effektiver durchmischt.

Mit einer Weiterbildung der vorliegenden Erfindung kann des Weiteren vorgesehen sein, dass der Pumpraum der Druckpumpe flüssigkeitsdicht ist und im Inneren der Druckpumpe angeordnet ist, wobei der Kolben über das Bedienelement manuell in zumindest einer Richtung antreibbar ist, so dass durch die Bewegung des Kolbens der Pumpraum zu verkleinern ist und mit dem sich dadurch verringernden Volumen des Pumpraums der Inhalt des Pumpraums, insbesondere die Monomerflüssigkeit im Pumpraum, durch die Verbindungsleitung in den Innenraum der zumindest einen Kartusche drückbar ist.

Hiermit wird eine besonders gute Pumpwirkung der Druckpumpe erreicht. Die Monomerflüssigkeit kann so besonders effektiv und durch Bedienen des Bedienelements aus dem Pumpraum in den Innenraum der Kartusche getrieben werden.

Dabei kann vorgesehen sein, dass die Volumenverkleinerung des Pumpraums bei einem vollen Hub des Kolbens mindestens so groß ist wie das Volumen der in den Innenraum der Kartusche einzupressenden Monomerflüssigkeit, bevorzugt die Volumenverkleinerung des Pumpraums bei einem vollen Hub des Kolbens mindestens so groß ist wie die Summe des Volumens der in den Innenraum der Kartusche einzupressenden Monomerflüssigkeit und der Verbindungsleitung.

Hierdurch wird sichergestellt, dass die Druckpumpe eine ausreichende beziehungsweise genau die passende und vorbestimmte Menge der Monomerflüssigkeit in den Innenraum der Kartusche transferiert. Bevorzugt geschieht dies bereits mit einem Hub des Kolbens.

Des Weiteren kann vorgesehen sein, dass der Kolben in einem Hohlzylinder axial beweglich gelagert ist, wobei der Hohlzylinder auf einer dem Kolben gegenüberliegenden ersten Seite geschlossen ist oder bis auf eine Durchführung für eine mit dem Bedienelement und dem Kolben verbundene Stange oder verbundenen Kabel geschlossen ist, insbesondere durch einen Verschluss geschlossen ist, wobei vorzugsweise der Pumpraum in dem Hohlzylinder zwischen dem Kolben und der ersten geschlossenen Seite gebildet ist, wobei besonders bevorzugt der Hohlzylinder auf einer zweiten Seite offen ist.

Bevorzugt haben der Pumpraum und der Kolben eine zylindrische Geometrie. Durch diese Maßnahmen und auch durch die zylindrische Geometrie wird eine besonders einfach und kostengünstig zu fertigende Druckpumpe vorgeschlagen, die leicht zu bedienen ist und die besonders unanfällig für Fehlfunktionen ist.

Bei Mischvorrichtungen mit Hohlzylinder kann des Weiteren vorgesehen sein, dass der Kolben der Druckpumpe über ein flexibles Kabel und/oder eine Stange anzutreiben ist, wobei ein erster Teil des Kabels und/oder die Stange durch den Pumpraum läuft und auf der ersten Seite durch eine flüssigkeitsdichte Durchführung aus dem Pumpraum herausgeführt ist, insbesondere durch eine flüssigkeitsdichte Durchführung durch den Verschluss aus dem Pumpraum herausgeführt ist, wobei der Kolben durch Bedienen des Bedienelements mit dem flexiblen Kabel und/oder der Stange in Richtung in Richtung der ersten Seite des Hohlzylinders zu ziehen ist, so dass sich der Pumpraum verkleinert.

Hierdurch kann der Kolben mit großer Kraft zur Verkleinerung des Pumpraums auf die gegenüberliegende erste Seite gezogen werden. Zugkräfte sind insbesondere über flexible Kabel einfacher zu vermitteln, so dass der Monomertransfer aus dem Pumpraum mit großer Kraft erfolgen kann und dadurch die Monomerflüssigkeit mit großem Druck in den Innenraum der Kartusche gedrückt werden kann, wodurch die Verteilung der Monomerflüssigkeit in dem Innenraum der Kartusche beziehungsweise im Zementpulver verbessert wird. Da der Monomertransfer bevorzugt mit einem einzigen Hub beziehungsweise Zug des Kolbens erfolgen soll, muss der Kolben mit dem Kabel nicht wieder rückführbar sein. Es kann sogar bevorzugt vorgesehen sein, dass der Kolben nur unidirektional anzutreiben ist.

Es wird auch vorgeschlagen, dass der Kolben über eine Stange und/oder ein Kabel mit dem Bedienelement verbunden oder verbindbar ist und vorzugsweise der Kolben durch Bedienen des Bedienelements in der Druckpumpe zu bewegen ist.

Hierdurch wird eine besonders einfache Mischvorrichtung bereitgestellt, bei der keine große Gefahr für mögliche Störungen besteht. Die direkte Verbindung des Bedienelements mit dem Kolben über das Kabel und/oder die Stange kann mit einem einteiligen Spritzgussteil aus Kunststoff realisiert werden. Alternativ kann auch eine Übersetzung beziehungsweise ein Getriebe vorgesehen sein, mit dem die Kraft, die auf das Bedienelement ausgeübt wird, zum Kolben übersetzt wird, um eine kräftigere Bewegung des Kolbens zu ermöglichen.

Bei einer Weiterentwicklung der Mischvorrichtung kann vorgesehen sein, dass in dem Innenraum der Kartusche ein beweglicher Austragskolben zum Austragen des gemischten Knochenzements aus der Kartusche angeordnet ist, wobei der Austragskolben vorzugsweise lösbar arretiert oder arretierbar ist, um eine Bewegung des Austragskolbens unter Einwirkung des Unterdrucks zu verhindern.

Mit dem Austragskolben wird die Bedienung der Mischvorrichtung vereinfacht.

Dabei kann vorgesehen sein, dass der Austragskolben einen Durchgang mit einer gasdurchlässige Porenscheibe aufweist, die für Zementpulver undurchlässig ist, wobei der Durchgang mit der Porenscheibe den Innenraum der Kartusche mit der Umgebung oder mit einem Vakuumanschluss gasdurchlässig verbindet, wobei der Durchgang gasdicht verschließbar ist, vorzugsweise mit einem Dichtungskolben des Austragskolbens gasdicht verschließbar ist.

Mit der Porenscheibe kann sichergestellt werden, dass der Innenraum der Kartusche mit dem Zementpulver darin mit Hilfe eines Gases wie Ethylenoxid sterilisiert werden kann, ohne dass die Gefahr besteht, dass das Zementpulver aus dem Innenraum der Kartusche nach außen in die Umgebung gelangt. Über den Vakuumanschluss kann im Innenraum der Kartusche ein Vakuum erzeugt werden, so dass der Inhalt des Mischraums unter Vakuum gemischt werden kann.

Bevorzugt kann auch vorgesehen sein, dass die Kartusche eine mit Zementpulver gefüllte Zementkartusche ist und in der Aufnahme ein separater Behälter enthaltend eine Monomerflüssigkeit angeordnet ist oder in dem integrierten Behälter eine Monomerflüssigkeit enthalten ist, wobei vorzugsweise die Aufnahme oder der integrierte Behälter durch ein zu öffnendes Trennelement flüssigkeitsundurchlässig mit dem Pumpraum der Druckpumpe verbunden ist.

Hiermit wird erreicht, dass die Monomerflüssigkeit aus dem separaten oder integrierten Behälter bereits in der Mischvorrichtung enthalten ist und mit einer Pumpbewegung der Druckpumpe beziehungsweise des Kolbens der Druckpumpe in den Innenraum der Kartusche überführt werden kann. Der Druck der mit der Druckpumpe manuell erzeugt wird, wird dabei zum Einpressen der Monomerflüssigkeit in die Kartusche genutzt. Insbesondere kann ein gegebenenfalls zu öffnendes Trennelement erfindungsgemäß mit dem Bedienelement geöffnet werden, vorzugsweise nachdem der integrierte Behälter oder der separate Behälter geöffnet ist.

Ferner kann vorgesehen sein, dass die Kartusche, die Druckpumpe und alle Leitungen sowie die Aufnahme oder der integrierte Behälter mit einem gemeinsamen Fußteil und/oder einem Gehäuse fest und/oder lösbar verbunden sind, wobei vorzugsweise die Druckpumpe und alle Leitungen sowie die Aufnahme oder der separate Behälter fest mit dem Fußteil und/oder dem Gehäuse verbunden sind und die Kartusche lösbar mit dem Fußteil und/oder dem Gehäuse verbunden ist.

Eine derartige Mischvorrichtung kann einfach aufgestellt werden und ist leicht bedienbar. Die Anwendung der Mischvorrichtung wird dadurch vereinfacht. Am Ort der Anwendung muss dann lediglich eine ebene Unterlage zum Aufstellen der Mischvorrichtung vorhanden sein, was in den meisten OP-Bereichen kein Problem darstellt.

Gemäß einer Ausführungsform kann vorgesehen sein, dass der separate Behälter enthaltend die Monomerflüssigkeit ein Folienbeutel ist, der in der Aufnahme mit der Öffnungseinrichtung aufschneidbar oder aufreißbar ist, oder eine Glasampulle ist, die in der Aufnahme mit der Öffnungseinrichtung aufbrechbar ist.

Hierdurch können handelsübliche Verpackungen der Monomerflüssigkeit eingesetzt werden, ohne dass diese außerhalb der Mischvorrichtung geöffnet werden müssen.

Es kann vorgesehen sein, dass unterhalb der Aufnahme oder des integrierten Behälters eine Verbindung zur Einleitung der Monomerflüssigkeit in den Pumpraum angeordnet ist.

Mit einer Weiterbildung der vorliegenden Erfindung wird ferner vorgeschlagen, dass durch die Bewegung des Kolbens in der Druckpumpe im Pumpraum ein Druck auf darin enthaltene Monomerflüssigkeit auszuüben ist, wobei mit dem Druck die Monomerflüssigkeit durch die Verbindungsleitung in den Innenraum der zumindest einen Kartusche zu drücken ist

Hiermit wird ein besonders einfacher und unanfälliger Aufbau bereitgestellt.

Erfindungsgemäß kann auch vorgesehen sein, dass die Druckpumpe aufgebaut ist mit einem Hohlzylinder, wobei der Hohlzylinder mit dem Innenraum der Kartusche verbunden oder verbindbar ist, einem flüssigkeitsdichten Verschluss an einem Hohlzylinderende, dem Kolben, der im Hohlzylinder flüssigkeitsdicht, axial beweglich angeordnet ist, wobei der Kolben in der Druckpumpe mit dem manuell bedienbaren Bedienelement bewegbar ist, wobei bei einem Bewegen des Kolbens mit dem manuell bedienbaren Bedienelement der Kolben axial in Richtung des Verschlusses bewegbar ist und dadurch eine Monomerflüssigkeit im Pumpraum in den Innenraum der Kartusche drückbar ist, wobei das Bedienelement mit der Öffnungseinrichtung in Wirkverbindung steht und wobei das Bedienelement mit der Mischeinrichtung im Innenraum der Kartusche derart verbunden ist, dass bei einem Bedienen des Bedienelements die Mischeinrichtung im Innenraum der Kartusche bewegbar ist.

Dieser Aufbau ist besonders einfach und die wesentlichen Teile hierfür können aus Kunststoff durch Spritzgießen gefertigt werden.

Mit einer besonders bevorzugten Ausführung der vorliegenden Erfindung kann vorgesehen sein, dass die Druckpumpe, die Öffnungseinrichtung und die Mischeinrichtung über die Bewegung des Bedienelements antreibbar sind, wobei bevorzugt die Bewegung des Bedienelements durch manuelle Krafteinwirkung erfolgt.

Hierdurch wird erreicht, dass die Mischvorrichtung keinerlei Energiespeicher und keine elektrischen oder elektronischen Antriebe benötigt. Dies ist deswegen erstrebenswert, da die Mischvorrichtung zum einmaligen Gebrauch bestimmt ist und auf diese Weise leichter zu recyceln ist. Zudem kann so erreicht werden, dass die Mischvorrichtung grundsätzlich anwendbar ist und keine Anschlüsse, wie Kabel oder Druckgasschläuche benötigt, um eingesetzt werden zu können.

Die der vorliegenden Erfindung zugrundeliegenden Aufgaben werden auch gelöst durch ein Verfahren zur Vermischung von Polymethylmethacrylat-Knochenzement in einem Innenraum einer Kartusche einer Mischvorrichtung, insbesondere einer solchen, oben beschriebenen Mischvorrichtung, bei dem ein Bedienelement bedient wird und dadurch ein integrierter Behälter der Mischvorrichtung oder ein separater Behälter, der in einer Aufnahme der Mischvorrichtung angeordnet ist, geöffnet wird, wobei anschließend eine in dem integrierten Behälter oder dem separaten Behälter enthaltene Monomerflüssigkeit als erste Komponente des Knochenzements in einen Pumpraum einer Druckpumpe fließt, durch eine anschließende weitere Bedienung des Bedienelements eine Bewegung eines Kolbens der Druckpumpe der Mischvorrichtung angetrieben wird, wobei durch die Bewegung des Kolbens die Monomerflüssigkeit aus dem Pumpraum der Druckpumpe durch eine Verbindungsleitung in den Innenraum der Kartusche gedrückt wird, wobei sich in dem Innenraum der Kartusche bereits ein Knochenzementpulver als zweite Komponente des Knochenzements befindet, und durch die Bedienung des Bedienelements eine Mischeinrichtung im Innenraum der Kartusche bewegt wird und durch die Bewegung der Mischeinrichtung ein Knochenzementteig im Innenraum der Kartusche aus dem Zementpulver und der Monomerflüssigkeit gemischt wird.

Dabei kann vorgesehen sein, dass das Volumen eines Pumpraums der Druckpumpe durch die manuelle Bewegung des Kolbens verkleinert wird und durch den dadurch entstehenden Druck die Monomerflüssigkeit in den Innenraum der Kartusche gedrückt wird.

Hiermit kann die Druckpumpe auf einfache Weise realisiert werden.

Ferner kann erfindungsgemäß vorgesehen sein, dass ein Zementpulver in dem Innenraum der Kartusche enthalten ist, eine Monomerflüssigkeit in den Innenraum der Kartusche eingeleitet wird, wobei Gas aus dem Innenraum der Kartusche dabei durch Einleiten der Monomerflüssigkeit durch die Porenscheibe und den Vakuumanschluss herausgedrückt, beziehungsweise verdrängt wird, und die Monomerflüssigkeit mit dem Zementpulver in dem evakuierten Innenraum der Kartusche durch eine Bewegung der Mischeinrichtung gemischt wird.

Durch die genannte Kombination und Wechselwirkung der Verfahrensschritte kann ein besonders einfacher und sicher zu realisierender Aufbau erreicht werden.

Des Weiteren kann vorgesehen sein, dass der Kolben der Druckpumpe mit dem Bedienelement bewegt wird, wodurch die in dem Pumpraum enthaltene Monomerflüssigkeit durch eine Verbindungsleitung in den Innenraum der Kartusche gedrückt wird, anschließend durch Bedienen desselben Bedienelements die Mischvorrichtung im Innenraum der Kartusche bewegt wird und dabei das Zementpulver mit der Monomerflüssigkeit vermischt wird, anschließend die Kartusche mit dem gemischten Zementteig entnommen wird und durch axiale Bewegung eines Austragskolbens der Zementteig aus der Kartusche heraus gepresst wird.

Hiermit wird das Verfahren dahingehend komplettiert, dass am Ende eine Zementkartusche mit einem unter Vakuum gemischten Knochenzementteig bereitgestellt wird, der unmittelbar angewendet werden kann.

Schließlich kann auch vorgesehen sein, dass das Zementpulver in der Kartusche angeordnet ist, die Monomerflüssigkeit in einer von der Kartusche separaten Aufnahme angeordnet ist, wobei die Monomerflüssigkeit in einem integrierten Behälter oder in einem separaten Behälter, vorzugsweise in einer Glasampulle in der Aufnahme, enthalten ist, der integrierte Behälter oder der separate Behälter durch Bedienen des Bedienelements und einer daraus resultierenden Bewegung der Öffnungseinrichtung geöffnet wird und die Monomerflüssigkeit aus dem Behälter in den Pumpraum fließt, bevor der Kolben durch ein weiteres Bedienen des Bedienelements angetrieben wird, und danach der Kolben axial in einem Hohlzylinder bewegt wird, wodurch die im Pumpraum befindliche Monomerflüssigkeit durch die Verbindungsleitung in den Innenraum der Kartusche gedrückt wird.

Dadurch wird das Verfahren weiter komplettiert.

Erfindungsgemäße Verfahren können auch durch die bestimmungsgemäße Anwendung oder Verwendung von Bauteilen erfindungsgemäßer Mischvorrichtungen gekennzeichnet sein.

Der Erfindung liegt die überraschende Erkenntnis zugrunde, dass es mit einem einzigen Bedienelement gelingt, sowohl die Druckpumpe und Mischeinrichtung zu betreiben beziehungsweise anzutreiben, als auch die Öffnungseinrichtung zu bedienen. Dies hat den Vorteil, dass keine komplizierten Handlungsanweisungen an das bedienende Personal notwendig sind. Durch Bedienen des einzigen Bedienelements können alle Abläufe gesteuert und angetrieben werden. Die Mischvorrichtung wird so maximal vereinfacht. Gleichzeitig sind keine Energiespeicher zum Antrieb notwendig und es bedarf keiner elektrischen beziehungsweise elektronischen Steuerung, um die Druckpumpe, die Mischeinrichtung und die Öffnungseinrichtung anzutreiben und zu steuern.

Gleichzeitig gelingt es mit Hilfe der manuell anzutreibenden Druckpumpe, eine von inneren und äußeren Energiequellen und anderen Versorgungsleitungen unabhängige Mischvorrichtung bereitzustellen. Die erfindungsgemäße Mischvorrichtung kann kompakt, leicht und platzsparend aufgebaut werden. Die Druckpumpe, die Öffnungseinrichtung und die gesamte Mischvorrichtung können mit einfachsten Mitteln aufgebaut werden, so dass die gesamte Mischvorrichtung als Einmalsystem verwendet werden kann. Eine zusätzliche Vakuumpumpe oder die geeignet modifizierte Druckpumpe kann ferner und erfindungsgemäß bevorzugt auch dazu eingesetzt werden, um den Innenraum der Kartusche zu evakuieren. Die beiden Komponenten des PMMA-Knochenzements können dann im Vakuum beziehungsweise in dem Unterdruck gemischt werden.

In Zementiersystemen nach der vorliegenden Erfindung ist eine Vorrichtung zur Überführung der Monomerflüssigkeit in den Innenraum der Kartusche enthalten, die zur temporären Erzeugung eines Drucks zum Transport einer flüssigen Monomerkomponente des Polymethylmethacrylat-Knochenzements geeignet ist.

Die der Erfindung zugrundeliegende Idee basiert auf der Erkenntnis, dass nur eine relativ geringe Energiemenge und damit ein geringer manueller Kraftaufwand notwendig ist, um den Behälter für die Monomerflüssigkeit zu öffnen, um die Monomerkomponente in den Innenraum der Kartusche zu überführen und um die Mischeinrichtung zum Durchmischen des Knochenzementteigs im Innenraum der Kartusche zu bewegen. Diese geringe Energiemenge kann ohne weiteres durch Bedienen eines Hebels als Bedienelement aufgebracht werden. Hierdurch ist die Mischvorrichtung einfach in der Handhabung und leicht zu bedienen sowie unabhängig von inneren und äußeren Energiespeichern. Durch einen geeigneten Aufbau lässt sich auch die Reihenfolge der Abläufe steuern, nämlich dass zuerst der Monomerbehälter geöffnet wird und erst anschließend die Druckpumpe und die Mischeinrichtung angetrieben werden.

Die Idee der Erfindung beruht auch darauf, dass durch manuelle Betätigung eines Bedienelements mit einem damit verbundenen Kolben in einem Hohlzylinder der Druckpumpe ein Überdruck in dem Hohlzylinder der Druckpumpe erzeugt wird, wobei sich die Monomerflüssigkeit über ein Leitungsmittel in den Innenraum der Kartusche gedrückt wird, in dem sich Zementpulver befindet. Danach erfolgt eine manuelle Vermischung der Zementkomponenten mit Hilfe einer Mischeinrichtung, die über dasselbe Bedienelement zeitgleich anzutreiben ist.

Beispielsweise kann die Erfindung mit dem folgenden Verfahren umgesetzt werden, bei dem durch Aktivierung mit einem manuell zu bedienenden Hebel oder allgemeiner einem manuell zu bedienenden Bedienelement die folgenden Funktionen in der Mischvorrichtung ablaufen:
1. Schritt: Betätigung des Handhebels beziehungsweise des Bedienelements und Brechen der Ampulle beziehungsweise Ampullen, Auslaufen der Monomerflüssigkeit innerhalb von 1 bis 2 Sekunden in einen Hohlzylinder, beim Anschlagpunkt des Hebels Einrasten einer elastischen Stange in ein Gegenrastmittel des Hebels, wobei die Stange in einen ersten Teil und einen zweiten Teil gegabelt ist;
2. Schritt: Rückwärtsbewegung des Handhebels beziehungsweise des Bedienelements in die Ausgangsposition, wobei ein Kolben durch die elastische Stange im Hohlzylinder bewegt wird, wobei die Monomerflüssigkeit über ein Leitungsmittel und eine Düse in das Zementpulver gepresst wird, Aushaken des Kolbens von einem ersten Teil der elastischen Stange, Bewegung eines zweiten Teils der elastischen Stange, das mit einer ersten Hülse drehbar verbunden ist, die mindestens eine äußerte Nocke besitzt, die in einem Steilgewinde einer zweiten Hülse beweglich angeordnet ist, das ein elastischer Rührstab (als Mischwelle) mit der ersten Hülse fest verbunden ist, so dass bei axialer Bewegung der ersten Hülse durch die zweite Hülse (beziehungsweise in der zweiten Hülse) die erste Hülse durch Eingriff der Nocke in das Steilgewinde gedreht wird, wodurch der Rührstab und rotiert und sich axial in der Kartusche bewegt;
3. Schritt: Weitere Betätigung des Handhebels beziehungsweise des Bedienelements, Bewegung der ersten Hülse in der zweiten Hülse, axiale Bewegung des Rührstabs in der Kartusche unter Drehung um die Längsachse;
4. Schritt: Wiederholung des 3. Schritts bis der Zementteig homogen gemischt ist;
5. Schritt: Ablösen der Kartusche beziehungsweise des Kartuschensystems durch Herausschrauben und Herausziehen des Mischstabs (der Mischwelle) mit dem Mischelement, unter Umklappen der Mischelemente (der Mischflügel der Mischeinrichtung).

Der wesentliche Vorteil der Erfindung ist, dass ein Prepack-Mischsystem vorgeschlagen wird, das in einfachster Weise, ohne spezielle Schulungsmaßnahmen, durch den medizinischen Anwender durch einfach manuelle Betätigung genutzt kann, um innerhalb von wenigen Sekunden (etwa innerhalb von 40 Sekunden) einen Polymethylmethacrylat-Knochenzementteig herzustellen. Vorteilhaft ist weiterhin, dass auf Grund der maximal vereinfachten Bedienung Anwenderfehler minimiert werden und dadurch die Patientensicherheit verbessert wird.

Die erfindungsgemäße Mischvorrichtung kann im Wesentlichen kostengünstig mit einfachen, durch Kunststoffspritzgießen herzustellenden Kunststoffteilen realisiert werden. Der besondere Vorteil der erfindungsgemäßen Vorrichtung besteht darin, dass die Vorrichtung ohne äußere Hilfsmittel, wie durch Druckluft angetriebene Vakuumpumpen und Vakuumschläuche, und ohne Energiequellen, wie Druckluft oder Batterien, betrieben werden kann. Die erfindungsgemäße Mischvorrichtung ist autonom verwendbar und kann selbst unter einfachsten beziehungsweise schwierigsten Operationsbedingungen verwendet werden. Mit der erfindungsgemäßen Mischvorrichtung wird ein geschlossenes Full-Prepack-Zementiersystem für preissensitive Märkte zur Verfügung gestellt.

Im Folgenden werden weitere Ausführungsbeispiele der Erfindung anhand von acht schematisch dargestellten Figuren erläutert, ohne jedoch dabei die Erfindung zu beschränken. Dabei zeigt:
- Figur 1:: eine schematische perspektivische Ansicht einer erfindungsgemäßen Mischvorrichtung;
- Figur 2:: eine schematische perspektivische Ansicht der Mischvorrichtung nach Figur 1 mit geöffnetem Gehäuse;
- Figur 3:: eine schematische perspektivische Querschnittansicht der Mischvorrichtung nach den Figuren 1 und 2 im Ausgangszustand;
- Figur 4:: eine schematische Querschnittansicht der Mischvorrichtung nach den Figuren 1 bis 3 im Ausgangszustand;
- Figur 5:: eine schematische Querschnittansicht der Mischvorrichtung nach den Figuren 1 bis 4 mit einer Schnittebene senkrecht zu dem Schnitt der Figuren 3 und 4;
- Figur 6:: eine schematische Querschnittansicht der Mischvorrichtung nach den Figuren 1 bis 5 während der Bedienung mit aufgebrochener Glasampulle;
- Figur 7:: eine schematische Querschnittansicht der Mischvorrichtung nach den Figuren 1 bis 6 während der Bedienung mit eingerastetem Kabel oder eingerasteter Stange; und
- Figur 8:: eine schematische Querschnittansicht der Mischvorrichtung nach den Figuren 1 bis 7 während der Bedienung beim Pumpen und Mischen.

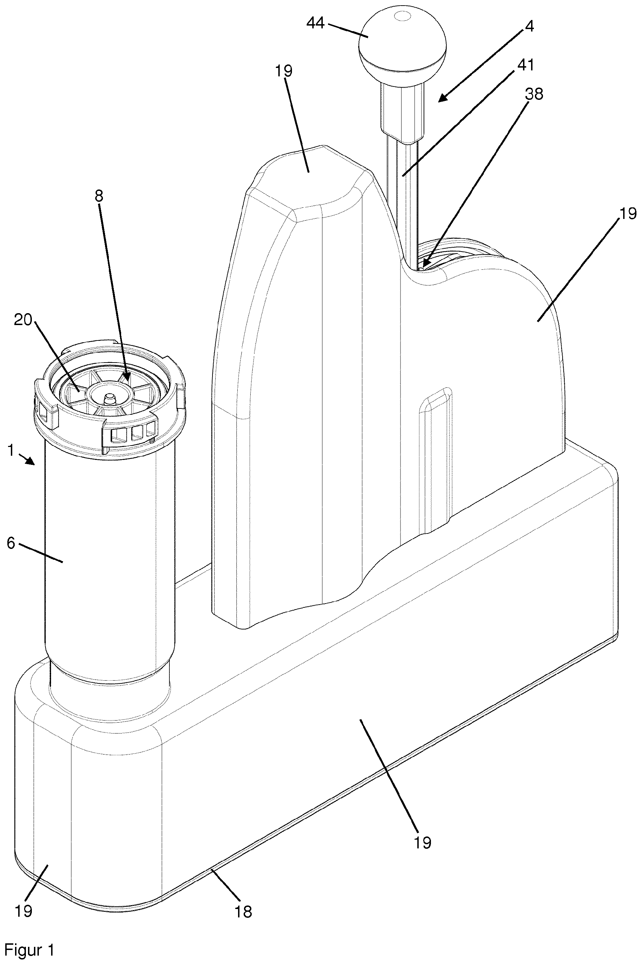

Die Figuren 1 bis 8 zeigen verschiedene Ansichten einer erfindungsgemäßen Mischvorrichtung vor und während des Betriebs. Die Mischvorrichtung besteht im Wesentlichen aus fünf Teilen, nämlich einem Kartuschensystem 1, einem Flüssigkeitsbehälter 2, einer manuell anzutreibenden Druckpumpe 3, einem Bedienelement 4 und einer Öffnungseinrichtung 5.

Zentraler Bestandteil des Kartuschensystems 1 ist eine Kartusche 6 mit einem zylindrischen Innenraum, die an ihrer Oberseite durch einen zweiteiligen Austragskolben 8 verschlossen ist, der im zylindrischen Innenraum der Kartusche 6 in Längsrichtung beweglich angeordnet ist. Die Kartusche 6 hat also einen zylindrischen Innenraum mit kreisförmiger Grundfläche. Die Kartusche 6 enthält ein Zementpulver 9 als Ausgangskomponente für einen Knochenzement.

Im Innenraum der Kartusche 6 ist ferner eine Mischeinrichtung 10 mit zwei oder mehr Mischflügeln 10 angeordnet, wobei die Mischeinrichtung 10 drehbar und in Längsrichtung verschiebbar im Innenraum der Kartusche 6 gelagert ist und die an einer Mischwelle 12 oder an einem Kabel 12 befestigt ist, die oder das drehbar und in Längsrichtung verschiebbar durch eine Durchführung in der Unterseite der Kartusche 6 in den Innenraum der Kartusche 6 geführt ist. Die Durchführung ist hierzu druckdicht ausgeführt. Die Mischwelle 12 kann auch als flexible Stange 12 ausgeführt werden.

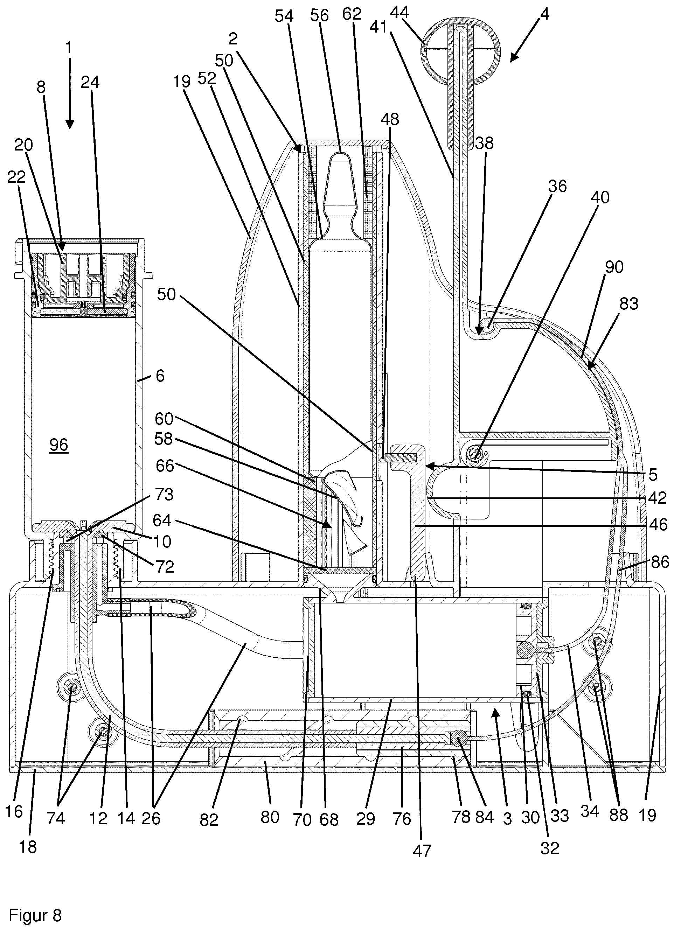

Das Kartuschensystem 1 ist mit dem Flüssigkeitsbehälter 2 und der Druckpumpe 3 über ein Fußteil 18 und ein Gehäuse 19 verbunden. Der Flüssigkeitsbehälter 2, die Druckpumpe 3, ein Teil des Bedienelements 4 und die Öffnungseinrichtung 5 sind von dem Gehäuse 19 umschlossen, wobei ein Teil des Bedienelements 4 aus dem Gehäuse 19 herausragt, während das Kartuschensystem 1 auf das Gehäuse 19 aufgeschraubt ist. Die Kartusche 6 endet an ihrer Unterseite in einem Stutzen mit einem Innengewinde 14, das auf ein Außengewinde 16 an einem Stutzen des Gehäuses 19 geschraubt ist. Das Fußteil 18 bildet dabei den Standfuß 18 der kompakten Mischvorrichtung. Die Kartusche 6 ist dadurch von dem Gehäuse 19 und damit dem Rest der Mischvorrichtung lösbar. Wenn der Knochenzement 96 (siehe Figur 8) mit der Mischvorrichtung im Innenraum der Kartusche 6 fertig gemischt ist, kann also die Kartusche 6 vom Gehäuse 19 abgeschraubt werden und in das Innengewinde 14 kann ein Austragsrohr (nicht gezeigt) eingeschraubt werden, durch das der fertige Knochenzementteig 96 (siehe Figur 8) durch Vortreiben des Austragskolbens 8 in Richtung des Innengewindes 14 ausgetrieben werden kann. Im Austragsrohr kann ein statischer Mischer vorgesehen sein, der eine zusätzliche Durchmischung des Knochenzementteigs 96 bewirkt.

Der zweiteilige Austragskolben 8 hat einen Dichtungskolben 20 und einen Sterilisationskolben 22. Der Sterilisationskolben 22 weist eine Membran oder Porenscheibe 24 auf, die für ein sterilisierendes Gas durchlässig ist aber für das Zementpulver 9 nicht durchlässig ist. Der Sterilisationskolben 22 wird nach dem Einfüllen des Zementpulvers 9 in die Kartusche 6 eingesetzt und schließt den Innenraum der Kartusche 6 nach außen ab. Anschließend kann der Inhalt der Kartusche 6 durch die gasdurchlässige Membran oder Porenscheibe 24 mit Ethylendioxid sterilisiert werden.

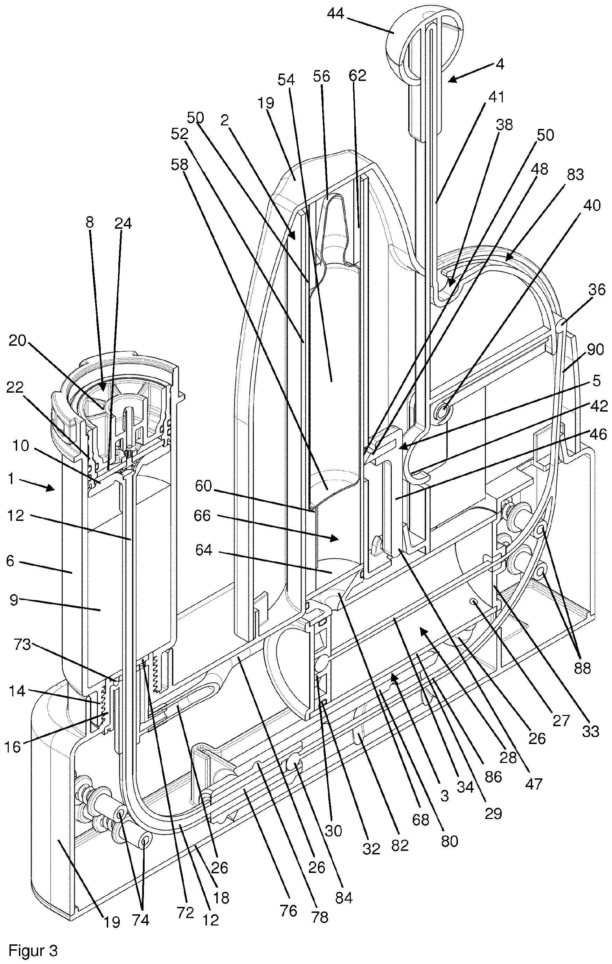

Der Dichtungskolben 20 kann in den Sterilisationskolben 22 gedrückt und mit diesem gas- und druckdicht verbunden werden. Die aneinander befestigten Kolbenteile 20, 22 bilden dann zusammen den Austragskolben 8, mit dem der Inhalt der Kartusche 6 durch die Öffnung im Stutzen mit dem Innengewinde 14 herausgepresst werden kann. Zunächst ist der Sterilisationskolben 22 auf der mit der Öffnung in dem Stutzen mit dem Innengewinde 14 gegenüberliegenden Seite (in den Figuren 3, 4 und 6 bis 8 oben) arretiert, wobei die Arretierung lösbar ist. Durch die Arretierung wird verhindert, dass sich der Sterilisationskolben 22 während der Sterilisation des Innenraums der Kartusche 6 sowie des Zementpulvers 9 ungewollt bewegt.

Über die Mischwelle 12 beziehungsweise das Kabel 12 können die Mischflügel 10 im Inneren der Kartusche 6, also im Innenraum der Kartusche 6 gedreht werden und in Längsrichtung der Kartusche 6 bewegt werden.

In dem Dichtungskolben 20 ist eine Durchführung vorgesehen, durch die der Innenraum der Kartusche 6 evakuiert werden kann. Hierzu kann eine Vakuumpumpe (nicht gezeigt) über eine Vakuumleitung (nicht gezeigt) als Teil der Mischvorrichtung an der Durchführung angeschlossen sein. Bevorzugt wird auch diese Vakuumpumpe mit dem Bedienelement 4 bedient und angetrieben. Der Dichtungskolben 20 schließt ansonsten druckdicht mit der Kartusche 6.

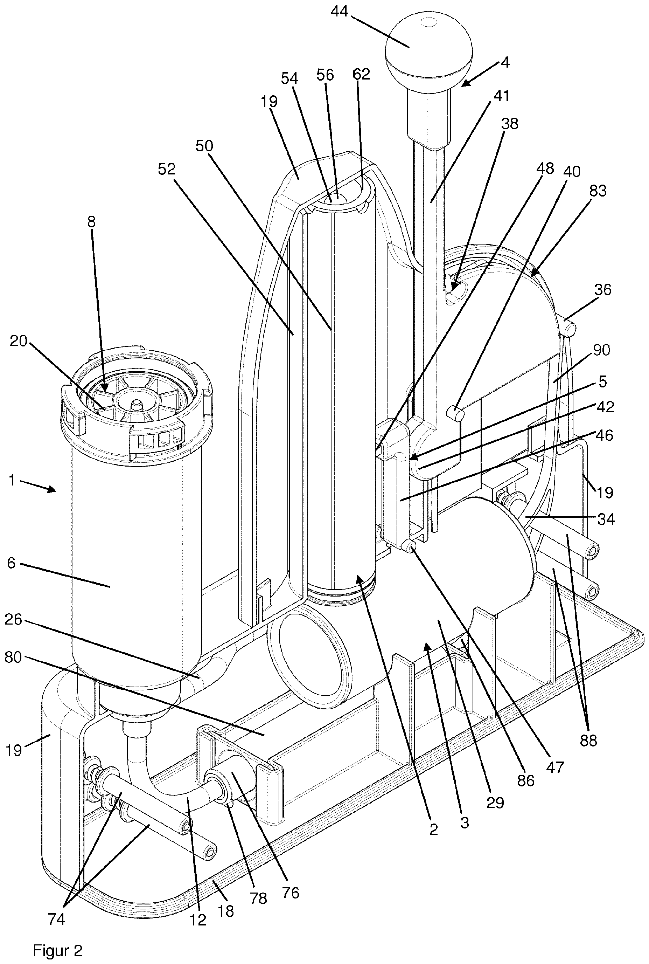

Im Boden der Kartusche 6 (in den Figuren 2 bis 4 und 6 bis 8 unten) mündet eine Verbindungsleitung 26 in den Innenraum der Kartusche. Die Verbindungsleitung 26 mündet über eine Einmündung 27 in einen Pumpraum 28 der Druckpumpe 3. Die Druckpumpe 3 weist einen stabilen Hohlzylinder 29 auf, der den zylindrischen Pumpraum 28 begrenzt. Der Hohlzylinder 29 ist über einen Kolben 30 druckdicht in zwei Teile getrennt. Hierzu weist der Kolben 30 eine umlaufende Dichtung 32 auf, die mit der Innenwand des Hohlzylinders 29 abschließt. Der Kolben 30 ist mit einem Kabel 34 oder einer flexiblen Stange 34 aus einem stabilen elastischen Kunststoff oder aus einem Metall wie Stahl verbunden, das durch eine Durchführung in einem rückseitigen Verschluss 33 führt. Der Verschluss 33, der Kolben 30 und der Hohlzylinder 29 begrenzen den Pumpraum 28. Auf der Vorderseite ist die Druckpumpe 3 durch einen vorderseitigen Verschluss verschlossen, in dem sich Öffnungen befinden, damit Luft nachströmen kann. Der vorderseitige Verschluss kann auch weggelassen werden und die Druckpumpe 3 auf dieser Seite völlig offen ausgeführt werden. Die Verbindungsleitung 26 ist bis zu der Einmündung 27 in die Druckpumpe 3 geführt, so dass die Durchführung in den Innenraum der Kartusche 6 über die Verbindungsleitung 26 flüssigkeitsdicht mit der Druckpumpe 3 genauer mit dem Pumpraum 28 der Druckpumpe 3 verbunden ist.

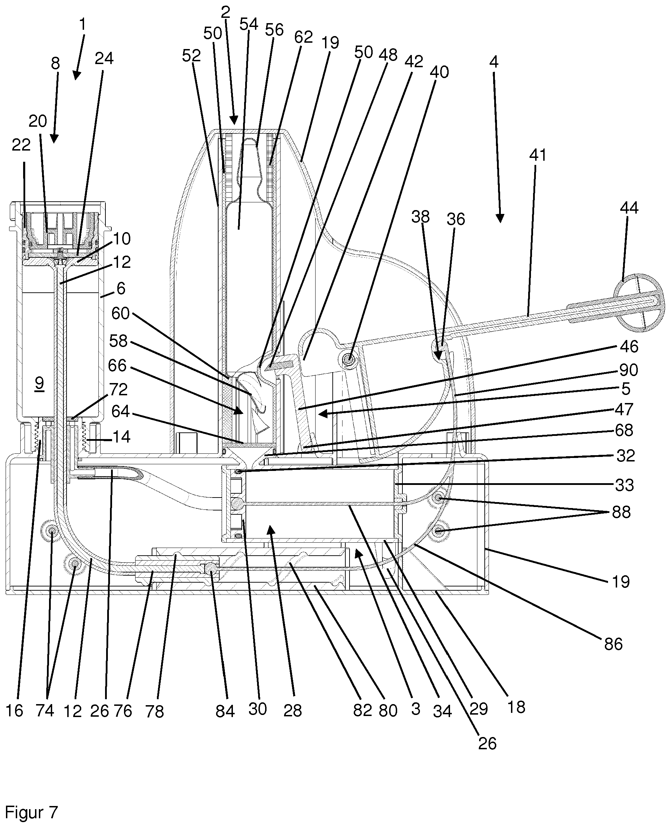

Das Kabel 34 ist mit einem Rastmittel 36 verbunden, das mit einem Gegenrastmittel 38 rasten kann (siehe Figuren 7 und 8). Das Gegenrastmittel 38 ist Teil des Bedienelements 4, das als ein um eine Achse 40 drehbarer beziehungsweise schwenkbarer Hebel 4 aufgebaut ist. Der Hebel 4 umfasst zwei Hebelarme 41, 42, die sich von der Achse 40 aus in unterschiedliche Richtungen erstrecken. Das eigentliche Bedienteil des Hebels 4 bildet der erste Hebelarm 41, der in einem Griff 44 endet, der von außen manuell bedienbar ist. Der erste Hebelarm 41 ragt also aus dem Gehäuse 19 heraus und der Griff 44 ist außerhalb des Gehäuses 19 angeordnet und kann vom Anwender der Mischvorrichtung manuell bedient werden. Dabei kann über den ersten Hebelarm 41 eine beträchtliche Kraft ins Innere der Mischvorrichtung übertragen werden, die zum Antreiben der Öffnungseinrichtung 5, der Druckpumpe 3 und der Mischeinrichtung 10 ausreicht und erfindungsgemäß angewendet wird.

Der zweite Hebelarm 42 drückt beim Schwenken des Hebels 4 beziehungsweise beim nach unten drücken des Hebels 4 auf die Öffnungseinrichtung 5 und treibt diese an. Dazu ist die Öffnungseinrichtung 5 mit einem Hebel 46 aufgebaut, der um eine Achse 47 drehbar beziehungsweise schwenkbar gelagert ist. An dem der Achse 47 gegenüberliegenden Ende des Hebels 46 ist ein Einsatz mit einer Kante 48 vorgesehen, die an der Aufnahme 2 beziehungsweise dem Flüssigkeitsbehälter 2 anliegt.

Der Flüssigkeitsbehälter 2 beziehungsweise die Aufnahme 2 umfasst einen inneren elastischen Einsatz 50, der beispielsweise aus einem Gummi bestehen kann, und einen starren Hohlzylinder 52 aus einem Kunststoff wie Plastik. In der Aufnahme 2 beziehungsweise dem Flüssigkeitsbehälter 2 ist eine Glasampulle 54 enthaltend eine Monomerflüssigkeit eingesteckt. Die Monomerflüssigkeit bildet mit dem Zementpulver 9 aus der Kartusche 6 einen Knochenzementteig 96, wenn sie miteinander durchmischt werden. Die Innenwandungen des elastischen Einsatzes 50 liegen an der Glasampulle 54 an. Die Glasampulle 54 weist einen Ampullenkopf 56 und einen dem Ampullenkopf 56 gegenüberliegenden Ampullenboden 58 auf. Die Glasampulle 54 sitzt mit dem Ampullenboden 58 auf einer Auflage 60 auf, die als Absatz 60 des elastischen Einsatzes 50 ausgeformt ist. Auf der Oberseite drückt ein Hohlzylinder 62 aus einem gasdurchlässigen Schaumstoff, der an der Innenseite des Gehäuses 19 befestigt ist, die Glasampulle 54 auf die Auflage 60. Zwischen dem Hohlzylinder 52 und dem Gehäuse 19 sind Öffnungen vorgesehen, durch die Luft oder Gas aus der Umgebung der Aufnahme 2 und der Mischvorrichtung in die Aufnahme 2 nachströmen kann. Dadurch kann die Monomerflüssigkeit leichter aus der Aufnahme 2 abfließen. Die Öffnungen zwischen dem Hohlzylinder 52 und dem Gehäuse 19 sind derart gestaltet, dass sie in der Zylindermantelwand des Hohlzylinders 52 angrenzend an die obere Grundfläche des Hohlzylinders 52 angeordnet sind. Der Hohlzylinder 52 liegt also nur bereichsweise an dem Gehäuse 19 an. Dazwischen kann Luft ins Innere der Aufnahme 2 nachströmen.

Im Bereich des Ampullenbodens 58 beziehungsweise der Auflage 60 weist der Hohlzylinder 52 eine Ausnehmung auf, innerhalb der die Kante 48 an dem elastischen Einsatz 50 anliegt, so dass der Ampullenboden 58 mit der Öffnungseinrichtung 5 abgebrochen und damit die Glasampulle 54 geöffnet werden kann. Der Ampullenkopf 56 der Glasampulle 54 wird üblicherweise zum Öffnen der Glasampulle 54 abgebrochen. Da die Glasampulle 54 am Hals dünn ausgeführt ist, führt dies jedoch dazu, dass die Monomerflüssigkeit aus der Glasampulle 54 nur langsam auslaufen kann und daher der Anwender warten muss, bis er die nächsten Schritte zum Bedienen der Mischvorrichtung durchführen kann. Dies ist bei dem weitgehend automatisierten Verfahren, das durch Bedienen des Hebels 4 beziehungsweise des Bedienelements 4 angetrieben wird nicht geeignet, da so nicht sichergestellt werden könnte, dass die Monomerflüssigkeit aus der Glasampulle 54 schon zur Verfügung steht, wenn die Druckpumpe 3 über das Bedienelement 4 angetrieben wird.

Die Glasampulle 54 steckt in dem Einsatz 50 aus dem verformbaren Material. Der Einsatz 50 bildet zusammen mit dem Hohlzylinder 52 die wesentlichen Teile der Aufnahme 2 für die Glasampulle 54. Die Glasampulle 54 kann aufgrund des Absatzes 60 nur bis zum Ampullenboden 58 in den Einsatz 50 des Flüssigkeitsbehälters 2 eingeschoben werden.

Der Flüssigkeitsbehälter 2 weist eine seitliche Öffnung auf, in der der Einsatz 50 eine verformbare Seitenwand bildet. An dieser Stelle kann die Glasampulle 54 geöffnet beziehungsweise aufgebrochen werden, indem ein Druck durch die verformbare Seitenwand 50 auf die Glasampulle 54 knapp oberhalb des Ampullenbodens 58 wirkt. Wenn der Ampullenboden 58 der Glasampulle 54 abgebrochen beziehungsweise die Glasampulle 54 geöffnet wird, kann die Monomerflüssigkeit aus der geöffneten Glasampulle 54 im vollen Querschnitt ausfließen, so dass die Monomerflüssigkeit schnell im vollen Umfang zur Weiterverarbeitung innerhalb der Mischvorrichtung zur Verfügung steht.

Um die verformbare Seitenwand 50 zu verformen und dadurch die Glasampulle 54 aufzubrechen, wird der Hebel 42 verwendet, der über den Hebel 4 bedienbar ist und der um die Achse 40 gedreht werden kann. Der Hebel 4 ist schwenkbar beziehungsweise drehbar um die Achse 40 gegen das Gehäuse 19 gelagert. Die Achse 40 teilt den Hebel 4 in einen langen Hebelarm 41, an dem der Griff 44 befestigt ist und einen kurzen Hebelarm 42 der innerhalb des Gehäuses 19 angeordnet ist. Zu Beginn kann der lange Hebelarm 41 nur von dem Flüssigkeitsbehälter 2 weg bewegt werden und nicht auf diesen zu, da der lange Hebelarm 41 oben an der Öffnung des Gehäuses 19 anliegt und so eine weitere Bewegung in diese Richtung unterbindet.

Der kurze Hebelarm 42 des Hebels 4 liegt auf seiner dem Flüssigkeitsbehälter 2 zugewandten Seite an dem Hebel 46 der Öffnungseinrichtung 5 an, der über ein Gelenk 47 beziehungsweise die Achse 47 drehbar um die Achse 47 mit dem Fußteil 18 beziehungsweise dem Gehäuse 19 der Mischvorrichtung verbunden ist. Dieser Hebel 46 der Öffnungseinrichtung 5 ist innerhalb des Gehäuses 19 angeordnet. Das freie Hebelende des Hebels 46 im Gehäuse 19 kann mit dem kurzen Hebelarm 42 bewegt werden. An der Spitze des freien Hebelendes ist die Kante 48 befestigt, die an der verformbaren Seitenwand 50 anliegt. Die Achse 47 des Hebels 46 ist dabei so angeordnet, dass sich das freie Hebelende und damit die Kante 48 in Richtung der verformbaren Seitenwand 50 und in Richtung des Fußteils 18 bewegt. Dadurch wird erreicht, dass die Kraft, die von der Kante 48 durch die verformbare Seitenwand 50 auf die Glasampulle 54 ausgeübt werden kann, die Glasampulle 54 auch leicht in Richtung des Absatzes 60 presst und somit die Glasampulle 50 in die Aufnahme 2 hineindrückt.

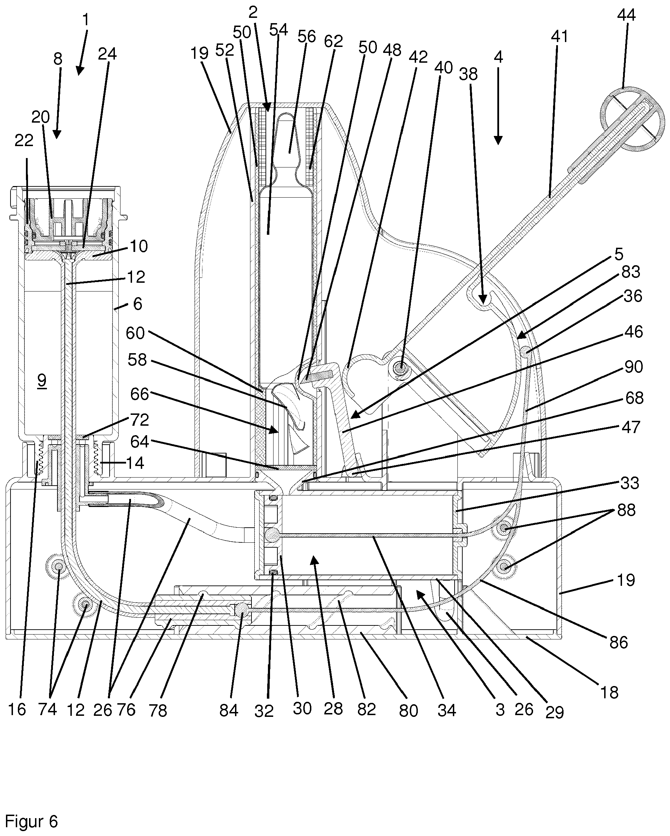

Unterhalb des Absatzes 60 ist ein Sieb 64 und/oder ein Filter 64 angeordnet, mit dem oder mit denen Glassplitter der geöffneten beziehungsweise aufgebrochenen Glasampulle 54 zurückgehalten werden. Der Abstand zwischen dem Absatz 60 und dem Sieb 64 und/oder Filter 64 ist größer als der Außendurchmesser der Glasampulle 54, so dass der abfallende Ampullenboden 58 sich in diesem Zwischenraum 66 drehen kann und den Ausfluss der Monomerflüssigkeit aus der geöffneten Glasampulle 54 nicht behindert (siehe Figuren 6 bis 8). Unterhalb des Siebs 64 und/oder Filters 64 ist ein Trichter 68 angeordnet, der in den Pumpraum 28 mündet, wenn der Kolben 30 an dem vorderseitigen Verschluss anliegt also gegenüber dem Verschluss 33 angeordnet ist. Die Monomerflüssigkeit fließt also nach dem Öffnen der Glasampulle 54 durch den Zwischenraum 66, das Sieb 64 und/oder den Filter 64 und den Trichter 68 in den Pumpraum 28.

Die Vorderseite der Kartusche 6 (in den Figuren 1 bis 4 und 6 bis 8 unten) ist durch das Fußteil 18 und das Gehäuse 19 über die Verbindungsleitung 26 flüssigkeitsdicht mit dem Zwischenraum 66 beziehungsweise dem Trichter 68 des Flüssigkeitsbehälters 2 verbunden.

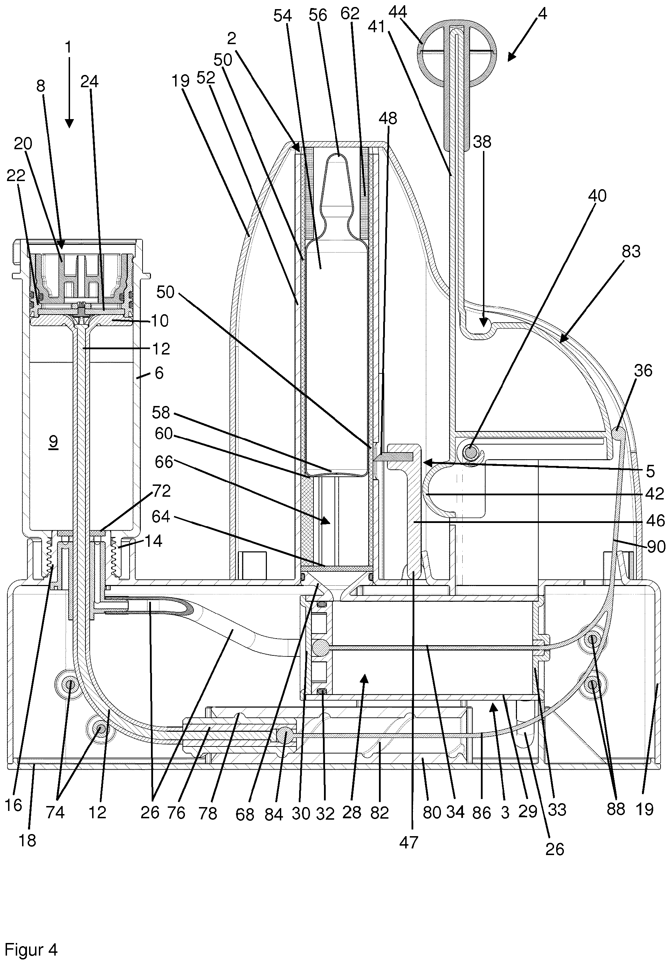

Die Kartusche 6 ist an dem Gehäuse 19 senkrecht lösbar befestigt. Die Verbindungsleitung 26 mündet in dem Stutzen mit dem Außengewinde 16 durch einen pulverundurchlässigen aber für die Monomerflüssigkeit durchlässigen Filter 72 in den Innenraum der Kartusche 6. Unterhalb des Filters 72 ist ein ringförmiger Kanal 73 ausgebildet (nur in den Figuren 3 und 8 gekennzeichnet aber auch in den Figuren 4, 6 und 7 zu erkennen), der zum Filter 72 hin offen ist, so dass der ebenfalls ringförmige Filter 72 den ringförmigen Kanal 73 abdeckt. Der ringförmige Kanal 73, in den die Verbindungsleitung 26 mündet und der genaugenommen noch zur Verbindungsleitung 26 gehört, und der ringförmige Filter 72 umschließen den Durchgang, in dem die Mischwelle 12 beziehungsweise das Kabel 12 in den Innenraum der Kartusche 6 geführt ist. Im Durchgang können dazu Abdichtungen (nicht gezeigt) oder zumindest Abstreifer (nicht gezeigt) vorgesehen sein. Mit dem ringförmigen Kanal 73 wird erreicht, dass die Monomerflüssigkeit durch den Filter 72 um die Mischwelle 12 herum in den Innenraum der Kartusche 6 eingeleitet wird. Es kann am Eingang der Verbindungsleitung 26 in den Innenraum der Kartusche 6 auch eine Düse (nicht gezeigt) vorgesehen sein, die die Monomerflüssigkeit im Innenraum beziehungsweise im Zementpulver 9 verteilt.

Der Flüssigkeitsbehälter 2 wird mit dem Gehäuse 19 nach oben verschlossen, nachdem die Glasampulle 54 in den Flüssigkeitsbehälter 2 eingesetzt wurde. Damit die Monomerflüssigkeit ohne Probleme aus der Glasampulle 54 und dem Zwischenraum 66 auslaufen beziehungsweise ablaufen kann, können zusätzlich in dem den Flüssigkeitsbehälter 2 abdeckenden Teil des Gehäuses 19 mehrere Durchgänge (nicht gezeigt) vorgesehen sein, durch die Luft von außerhalb in den Flüssigkeitsbehälter 2 nachströmen kann. Nach dem Aufbrechen der Glasampulle 54 fließt die Monomerflüssigkeit in dem Pumpraum 28 und kann mit dem Kolben 30 durch die Verbindungsleitung 26 in den Innenraum der Kartusche 6 gedrückt werden. Der dafür notwendige Druck wird mit der Druckpumpe 3 erzeugt, indem der Kolben 30 mit dem Kabel 34 oder der Stange 34 zum Verschluss 33 gezogen wird, wobei sich der Pumpraum 28 der Druckpumpe 3 verkleinert. Im Innenraum der Kartusche 6 kann die Monomerflüssigkeit dann mit dem Zementpulver 9 mit Hilfe der Mischeinrichtung 10 gemischt werden, um den Knochenzement 96 beziehungsweise einen Knochenzementteig 96 zu erzeugen. Die Mischung kann unter Vakuum beziehungsweise unter Unterdruck durchgeführt werden, indem an den Vakuumanschluss am Dichtungskolben 20 eine Vakuumpumpe (nicht gezeigt) oder eine andere Unterdruckquelle angeschlossen wird.

Die Mischeinrichtung 10 wird zum Durchmischen des Inhalts des Innenraums der Kartusche 6 verwendet. Das Kabel 12 beziehungsweise die Mischwelle 12, über die die Mischeinrichtung 10 im Innenraum gedreht wird und in Längsrichtung des Innenraums auf und ab bewegt wird, wird über Stifte 74 beziehungsweise Umlenkrollen 74 in Richtung eines Zylinders 76 umgelenkt. Die Umlenkrollen 74 können mit federnd gelagerten Röhrchen beziehungsweise Umlenkhülsen aufgebaut werden. Die Federn dienen dabei lediglich der Fixierung der Umlenkrollen 74 beziehungsweise Umlenkhülsen. Das Kabel 12 beziehungsweise die Mischwelle 12 ist starr mit dem Zylinder 76 verbunden. Der Zylinder 76 weist an der Außenseite ein steiles Außengewinde 78 auf. Der Zylinder 76 ist in einer Hülse 80 mit einem zum Außengewinde 78 passenden Innengewinde 82 angeordnet. Bei einer Bewegung des Zylinders 76 in der Hülse 80 in Längsrichtung (der Zylinderachse) wird die Mischeinrichtung 10 über das Kabel 12 beziehungsweise die Mischwelle 12 also in Längsrichtung des Innenraums der Kartusche 6 bewegt und gleichzeitig aufgrund der Gewinde 78, 82 um die Mischwelle 12 gedreht und damit der Inhalt des Innenraums durchmischt. Alternativ zum Außengewinde 78 an der Hülse kann auch ein oder mehrere Vorsprünge beziehungsweise eine oder mehrere Nocken 78 vorgesehen sein, die in dem Innengewinde 82 laufen und so den Zylinder 76 in der Hülse 80 drehen.

Der Zylinder 76 ist über ein Kugelgelenk beziehungsweise einen Kugelgelenkkopf 84 mit einem starren Kabel 86 oder einer flexiblen Stange 86, das analog dem Kabel 34 beziehungsweise der flexiblen Stange 34 für die Druckpumpe 3 aufgebaut ist, verbunden. Der Kugelgelenkkopf 84 kann sich also innerhalb einer Aufnahme für den Kugelgelenkkopf 84 des Zylinders 76 bewegen und darin drehen. Hierdurch wird ermöglicht, dass bei einer Bewegung des Kabels 86 gleichzeitig eine Drehung des Zylinders 76 in der Hülse 80 erzwungen wird. Das Kabel 86, das mit dem Zylinder 76 verbunden ist und das Kabel 34, das mit dem Kolben 30 der Druckpumpe 3 verbunden ist, sind miteinander verbunden, wobei beide über Stifte 88 beziehungsweise Umlenkrollen 88 positioniert werden. Die Umlenkrollen 88 sind analog den Umlenkrollen 74 aufgebaut. Ebenso kann die Verbindung des Kabels 34 zum Kolben 30 mit einem Kugelgelenk aufgebaut werden. Die beiden Kabel 34, 86 oder flexiblen Stangen 34, 86 schließen sich zu einem Kabel 90 oder einer flexiblen Stange 90 zusammen, das nach oben zum Hebel 4 beziehungsweise zum Bedienelement 4 geführt ist, wobei das Kabel 90 oder die flexible Stange 90 dort in dem Rastmittel 36 endet. Auch das Kabel 90 ist analog dem Kabel 34 für die Druckpumpe 3 aufgebaut, beziehungsweise die flexible Stange 90 analog der flexiblen Stange 34 für die Druckpumpe 3. Die gegabelt verbundenen Kabel 34, 86, 90 oder die gegabelte Stange 34, 86, 90 können aus einem Kunststoff durch Spritzguss gefertigt sein, beziehungsweise das gemeinsame gegabelte Kabel 34, 86, 90 oder die gegabelte Stange 34, 86, 90 kann aus einem Kunststoff durch Spritzguss gefertigt sein. Das Rastmittel 36 am Ende des Kabels 90 ist dabei so gelagert und derart vorgespannt, dass es in das Gegenrastmittel 38 greift und mit diesem rastet, wenn der Hebel 4 weit genug gedreht beziehungsweise geschwenkt wird, beziehungsweise wenn das Gegenrastmittel 38 auf die Höhe des Rastmittels 36 geschwenkt wird.

Der maximale Hub, der durch eine als Evolvente 83 geformte Abrundung am Hebel 4 bestimmt ist, indem das Kabel 90 oder die flexible Stange 90 nach erfolgter Rastung auf die Evolvente 83 aufgezogen wird, reicht dazu aus, dass der Innenraum der Kartusche 6 in voller Länge von der Mischeinrichtung 10 durchfahren werden kann. Dies ist in den Figuren 3, 4, 6 und 7 im Vergleich mit Figur 8 zu erkennen, da beim vollständigen Hub des Hebels 4, der in Figur 8 dargestellt ist, die Mischeinrichtung 10 beziehungsweise die Mischflügel 10 an der Vorderseite des Innenraums der Kartusche 6 auf dem Filter 72 anliegt, während ohne Hub, wie in Figur 7 dargestellt, die Mischeinrichtung 10 beziehungsweise die Mischflügel 10 auf der Rückseite des Innenraums der Kartusche 6 an dem Austragskolben 8, beziehungsweise dem Sterilisationskolben 22 und der Porenscheibe 24 anliegen. Hierdurch wird eine vollständige Durchmischung des Innenraums der Kartusche 6 mit der Mischeinrichtung 10 ermöglicht. Das Gegenrastmittel 38 ist hierzu an dem bezüglich der Zugrichtung der Kabel 90, 34, 86 oder der flexiblen Stange 90, 34, 86 abgewandten Ende der Evolvente 83 angeordnet, damit das Kabel 90 oder die flexible Stange 90 über einen weiten Bereich der Evolvente 83 aufgezogen werden kann.

Anstatt das Kabel 34 oder die flexible Stange 34 der Druckpumpe 3 und das Kabel 86 oder die flexible Stange 86 zum Zylinder 76 miteinander zu dem Kabel 90 oder der flexiblen Stange 90, an dem das Rastmittel 36 angeordnet ist, zu verbinden, kann genauso gut jedes der Kabel 34, 86 oder jede der flexiblen Stangen 34, 86 ein eigenes Rastmittel aufweisen, das in das Gegenrastmittel 38 oder das zwei unterschiedliche Gegenrastmittel am Ende der Evolvente 83 beziehungsweise dem Hebel 4 greift und mit diesem beziehungsweise diesen rastet.

In einer alternativen Ausführung einer erfindungsgemäßen Mischvorrichtung kann das Kabel 12 direkt mit dem Kabel 86 verbunden sein, beziehungsweise die beiden Kabel 12, 86 als ein gemeinsames durchgehendes Kabel ausgeführt sein, oder die flexible Stange 12 mit der flexiblen Stange 86 einteilig ausgeführt sein. Der Zylinder 76, die Hülse 80 sowie die Gewinde 78, 82 sind dann überflüssig und nicht vorhanden. Dies führt dann dazu, dass die Mischeinrichtung 10 nicht mehr durch die Mischwelle 12 im Innenraum der Kartusche 6 gedreht wird. Eine Durchmischung des Innenraums der Kartusche 6 wird dann nur noch durch die Auf- und Abwärtsbewegung der Mischeinrichtung 10 in Längsrichtung erreicht. Durch eine geeignete Neigung der Mischflügel 10 oder einiger Mischflügel 10 und/oder durch eine Führung zumindest eines Vorsprungs (nicht gezeigt) an der Mischeinrichtung 10 in wenigstens einer spiralförmigen Nut (nicht gezeigt) in der Innenwand der Kartusche 6 kann auch auf andere Weise eine Drehung der Mischeinrichtung 10 in der Kartusche 6 erzwungen werden, sofern nicht einfach auf die Drehung der Mischeinrichtung 10 in der Kartusche 6 verzichtet wird.

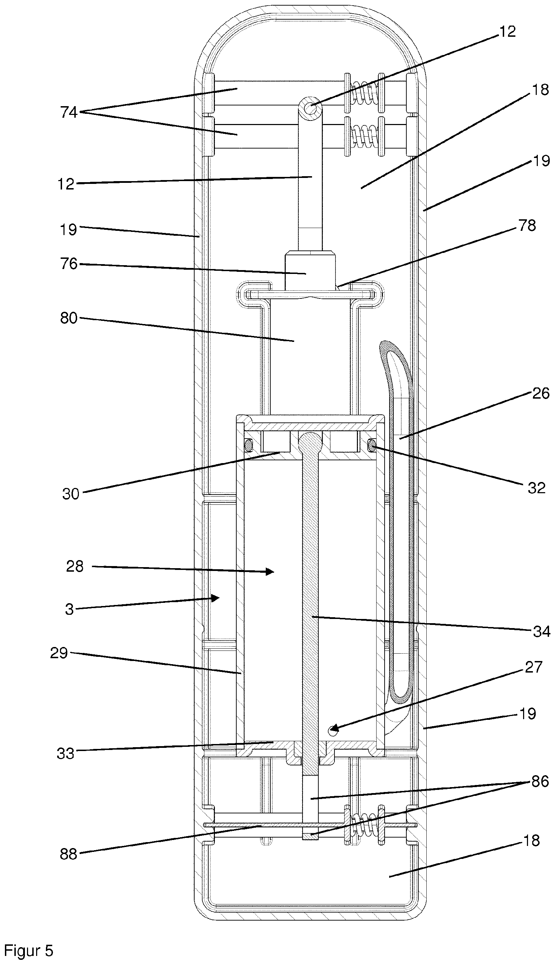

Figur 5 zeigt eine schematische Querschnittansicht der Mischvorrichtung nach den Figuren 1 bis 4 und 6 bis 8 mit einer Schnittebene senkrecht zu dem Schnitt der Figuren 3 und 4 sowie 6 bis 8.

Die Mischvorrichtung zeichnet sich erfindungsgemäß durch die Anwendbarkeit des folgenden beispielhaften erfindungsgemäßen Verfahrens aus. Die Monomerflüssigkeit wird in dem Flüssigkeitsbehälter 2 bereitgestellt, indem die Glasampulle 54 mit der Öffnungseinrichtung 5, wie oben geschildert, aufgebrochen wird. Dazu wird der Hebel 4, der sich ursprünglich in einer senkrechten Position (siehe Figur 1 bis 4) befindet nach unten gedrückt (siehe Figur 6). Während die Monomerflüssigkeit ausläuft und den Pumpraum 28 der Druckpumpe 3 füllt, wird der Hebel 40 weiter gedreht beziehungsweise geschwenkt, bis das Gegenrastmittel 38 auf die Höhe des Rastmittels 36 gedreht ist und beide miteinander rasten (siehe Figur 7). Durch das Rasten des Rastmittels 36 mit dem Gegenrastmittel 38 kann erst jetzt die Druckpumpe 3 mit dem Hebel 4 über die Kabel 34, 90 oder die flexible Stange 34, 90 bewegt beziehungsweise angetrieben werden und die Mischeinrichtung 10 mit dem Hebel 4 über die Kabel 86, 90 oder die flexible Stange 86, 90, den Zylinder 76 und das Kabel 12 beziehungsweise die Mischwelle 12 angetrieben werden. Dazu wird der Hebel 4 vom unteren Anschlag weg wieder in die Ausgangsposition (Figuren 2 bis 4) gedreht beziehungsweise geschwenkt.

Die Druckpumpe 3 wird verwendet, indem der Kolben 30 mit dem Bedienelement 4 über das Kabel 34, 90 beziehungsweise über die flexible Stange 34, 90 von dem vorderseitigen Verschluss weg in Richtung des Verschlusses 33 gezogen wird. Dabei verkleinert sich im Inneren der Druckpumpe 3 der Pumpraum 28. Durch die Verkleinerung des Pumpraums 28 wird die Monomerflüssigkeit aus dem Pumpraum 28 und die Verbindungsleitung 26 in den Innenraum der Kartusche 6 gedrückt.

Der Kolben 30 wird bis zum Ende des Hohlzylinders 29 (in den Figuren 3 bis 4 und 6 bis 8 rechts) bewegt. Diese Anordnung ist in Figur 8 gezeigt. Die Volumenabnahme des Pumpraums 28 kann vorzugsweise dazu ausreichen, um die Monomerflüssigkeit aus dem Flüssigkeitsbehälter 2 vollständig in den Innenraum der Kartusche 6 zu drücken, so dass bereits ein Hub des Kolbens 30 ausreicht, um die Monomerflüssigkeit in den Innenraum der Kartusche 6 zu transferieren. Bevorzugt kann der expandierte Pumpraum 28 zu diesem Zweck größer oder gleich groß dem Volumen der Monomerflüssigkeit in der Glasampulle 54 sein. Alternativ zur Überführung der Monomerflüssigkeit mit einem einzigen Hub des Kolbens 30 kann die Monomerflüssigkeit aber auch mit mehreren Hüben des Kolbens 30 durch wiederholtes Bedienen des Hebels 4 (hin und her schwenken des Hebels 4) in den Innenraum der Kartusche 6 gedrückt werden. Dazu muss der Kolben 30 etwas anders als in den Figuren 3 bis 8 gezeigt aufgebaut sein. Der Kolben 30 sollte dazu außer in der Ausgangsstellung (siehe Figuren 3 bis 7) die Verbindung zum Trichter 68 immer verschließen also zumindest in diesem Bereich verlängert sein. Damit wird dann verhindert, dass die Monomerflüssigkeit einfach in den freien Bereich neben dem Kolben 30 in die Druckpumpe 3 fließt.

Gleichzeitig mit der Bewegung des Kolbens 30 wird über die Kabel 86, 90 oder die Stange 86, 90 und das Kugelgelenk 84 der Zylinder 76 in der Hülse 80 in Längsrichtung bewegt und dabei über die Gewinde 78, 82 gedreht. Die Bewegung in Längsrichtung und die Drehung wird über die Mischwelle 12 beziehungsweise das Kabel 12 durch die Durchführung auf die Mischeinrichtung 10 im Innenraum der Kartusche 6 übertragen. Durch mehrfaches Schwenken des Hebels 4 in beide Richtungen und damit Bewegen der Mischeinrichtung 10 im Innenraum der Kartusche 6 wird der Inhalt, nämlich das Knochenzementpulver 9 und die eingedrückte Monomerflüssigkeit, durchmischt und dadurch ein Knochenzementteig 96 im Innenraum der Kartusche 6 erzeugt.

Wenn die Ausgangskomponenten im Innenraum der Kartusche 6 mit den Mischflügeln 10 gemischt wurden, wird das Kartuschensystem 1 von dem Gehäuse 19 beziehungsweise dem Außengewinde 16 des Gehäuses 19 abgeschraubt und das Kabel 12 beziehungsweise die Mischwelle 12 mit der Mischeinrichtung 10 aus dem Innenraum der Kartusche 6 herausgezogen. Dabei klappen die Mischflügel 10 nach oben zusammen. Hierfür sind an der Verbindung der Mischflügel 10 zu der Mischwelle 12 Material-Verjüngungen als Sollknickstellen vorgesehen.

Nachdem das Kartuschensystem 1 abgeschraubt wurde, wird in das Innengewinde 14 ein Austragsrohr (nicht gezeigt) mit einem passenden Außengewinde eingeschraubt, durch das der gemischte Knochenzement 96 appliziert werden kann. Der aus dem Sterilisationskolben 22 und dem Dichtungskolben 20 zusammengesetzte Förderkolben 8 oder Austragskolben 8 wird entrastet und kann mit einem Applikationsgerät (nicht gezeigt) ins Innere der Kartusche 6 getrieben werden. Dadurch wird der Inhalt der Kartusche 6, also der gemischte Knochenzementteig 96 aus der gegenüberliegenden Öffnung und durch das aufgeschraubte Austragsrohr ausgepresst.

Die Bauteile der Mischvorrichtung können bis auf die Glasampulle 54, die Filter 64, 72 und die Ausgangskomponenten des Knochenzements durch Spritzgießen aus Kunststoff gefertigt werden.

Die Verbindungsleitung 26 und die Kabel 34, 86, 90 oder die gegabelte Stange 34, 86, 90 sind in dem Gehäuse 19 aus Kunststoff angeordnet, das mit dem Fußteil 18 fest verbunden ist, wobei das Fußteil 18 einen flachen Boden aufweist, damit die Mischvorrichtung auf einer flachen Unterlage aufgestellt werden kann.

Anstatt der mit dem beschriebenen Ausführungsbeispiel verwendeten Glasampulle 54 kann auch ein anderer Monomerflüssigkeitsbehälter verwendet werden. Beispielsweise kann ein Folienbeutel enthaltend die Monomerflüssigkeit als Behälter für die Monomerflüssigkeit in eine modifizierte Aufnahme eingesetzt werden. Der Folienbeutel kann beispielsweise ein mit Aluminium beschichteter Kunststoffbeutel sein, der gegen die Monomerflüssigkeit chemisch ausreichend beständig ist. In der alternativen Aufnahme 2 kann dann ein Dorn oder besser eine Klinge vorgesehen sein, der oder die mit der Öffnungseinrichtung 5 gegen den Folienbeutel zu drücken und bewegen ist, so dass der Folienbeutel über die Öffnungseinrichtung 5 mit dem Dorn oder der Klinge aufzustechen oder aufzuschlitzen ist, so dass anschließend die Monomerflüssigkeit aus dem Folienbeutel ausläuft und in der Aufnahme 2 zur Verfügung steht. Ferner kann der Behälter für die Monomerflüssigkeit auch fest in der Aufnahme 2 und damit in die Mischvorrichtung integriert sein und sich mit der Öffnungseinrichtung 5 zu dem Filter 64 und/oder Sieb 64 beziehungsweise zum Trichter 68 hin öffnen lassen.

Die Variante mit Glasampulle 54 als Behälter für die Monomerflüssigkeit ist aber erfindungsgemäß bevorzugt, da die mit Monomerflüssigkeit gefüllten Glasampullen 54 kommerziell kostengünstig zu erhalten sind und zudem Glasampullen 54 zur langfristigen Lagerung der Monomerflüssigkeit besonders gut geeignet sind. Dabei ist es besonders bevorzugt, dass die Glasampulle 54 bereits in der Aufnahme 2 der Mischvorrichtung enthalten ist.

Mit der beschriebenen Mischvorrichtung können die beiden Ausgangskomponenten des Knochenzements gelagert und zu einem beliebigen späteren Zeitpunkt gemischt werden. Dabei muss die Mischvorrichtung an keine externe Versorgung (Strom, Wasser oder Druckgas) angeschlossen werden. Es ist kein interner Energiespeicher, wie eine Batterie, eine Druckgaspatrone oder eine gespannte Feder, zum Antreiben der Mischvorrichtung beziehungsweise der Druckpumpe 3, der Mischeinrichtung 10 und der Öffnungseinrichtung 5 notwendig. Die zum Überführen der Monomerflüssigkeit notwendige Energie wird ebenso manuell aufgebracht, wie die zum Öffnen der Glasampulle 54 und die zum Bewegen der Mischeinrichtung 10 notwendige Kraft.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Kartuschensystem
- 2: Aufnahme
- 3: Druckpumpe
- 4: Bedienelement / Hebel
- 5: Öffnungseinrichtung
- 6: Kartusche / Kartuschenwand / Hohlzylinder
- 8: Austragskolben
- 9: Zementpulver
- 10: Mischeinrichtung / Mischflügel
- 12: Mischwelle / Kabel
- 14: Innengewinde
- 16: Außengewinde
- 18: Fußteil / Standfuß
- 19: Gehäuse
- 20: Dichtungskolben
- 22: Sterilisationskolben
- 24: Porenscheibe
- 26: Verbindungsleitung / Vakuumleitung
- 27: Einmündung
- 28: Pumpraum
- 29: Hohlzylinder
- 30: Kolben
- 32: Dichtung / O-Ring
- 33: Verschluss
- 34: Kabel / flexible Stange
- 36: Rastmittel
- 38: Gegenrastmittel
- 40: Achse
- 41: Hebelarm
- 42: Hebelarm
- 44: Griff
- 46: Hebel
- 47: Achse
- 48: Kante
- 50: Elastischer Einsatz
- 52: Hohlzylinder
- 54: Glasampulle mit Monomerflüssigkeit
- 56: Ampullenkopf
- 58: Ampullenboden
- 60: Auflage / Absatz
- 62: Hohlzylinder
- 64: Filter / Sieb
- 66: Zwischenraum
- 68: Trichter
- 72: Pulverundurchlässiger und Flüssigkeitsdurchlässiger Filter
- 73: Ringförmiger Kanal
- 74: Stift / Umlenkrolle
- 76: Zylinder
- 78: Außengewinde / Nocke
- 80: Hülse
- 82: Innengewinde
- 83: Evolvente
- 84: Kugelgelenkkopf
- 86: Kabel / flexible Stange
- 88: Stift / Umlenkrolle
- 90: Kabel / flexible Stange
- 96: Knochenzementteig

## Patentansprüche

1. Mischvorrichtung zum Mischen von Polymethylmethacrylat-Knochenzement aus einer Monomerflüssigkeit und einem Zementpulver (9), die Mischvorrichtung aufweisend
zumindest eine Kartusche (6) umfassend einen evakuierbaren Innenraum zum Mischen des Knochenzements (96),
eine Mischeinrichtung (10) zum Durchmischen des Inhalts des Innenraums der zumindest einen Kartusche (6), die beweglich im Innenraum angeordnet ist,
eine Aufnahme (2) zur Aufnahme eines die Monomerflüssigkeit enthaltenden separaten Behälters (54) oder aufweisend einen integrierten Behälter enthaltend die Monomerflüssigkeit,
eine Öffnungseinrichtung (5), die im Bereich der Aufnahme (2) gegen die Aufnahme (2) beweglich angeordnet ist, so dass durch eine Bewegung der Öffnungseinrichtung (5) ein in der Aufnahme (2) angeordneter separater Behälter (54) mit der Öffnungseinrichtung (5) zu öffnen ist, oder die Öffnungseinrichtung (5) im Bereich des integrierten Behälters gegen den integrierten Behälter beweglich angeordnet ist, so dass durch eine Bewegung der Öffnungseinrichtung (5) der integrierte Behälter mit der Öffnungseinrichtung (5) zu öffnen ist,
eine Druckpumpe (3), in der ein beweglicher Kolben (30) zum Fördern einer Flüssigkeit angeordnet ist und der einen Pumpraum (28) der Druckpumpe (3) begrenzt, und
eine Verbindungsleitung (26), die den Innenraum der zumindest einen Kartusche (6) mit dem Pumpraum (28) der Druckpumpe (3) verbindet, wobei die Mischvorrichtung ein von außen bedienbares Bedienelement (4) aufweist, wobei mit dem Bedienelement (4) der Kolben (30) in der Druckpumpe (3) manuell bewegbar ist, und wobei
mit demselben Bedienelement (4) die Öffnungseinrichtung (5) gegen die Aufnahme (2) oder gegen den integrierten Behälter zu bewegen ist, und
mit demselben Bedienelement (4) die Mischeinrichtung (10) im Innenraum der Kartusche (6) zum Durchmischen des Inhalts des Innenraums der Kartusche (6) bewegbar ist.

2. Mischvorrichtung nach Anspruch 1, **dadurch gekennzeichnet, dass**
das Bedienelement (4) mit dem Kolben (30) derart verbunden oder verbindbar ist, dass der Kolben (30) in der Druckpumpe (3) durch Bedienen des Bedienelements (4) manuell bewegbar ist.

3. Mischvorrichtung nach einem der vorangehenden Ansprüche, **dadurch gekennzeichnet, dass**
die Öffnungseinrichtung (5) einen ersten Hebel (46) aufweist, der um eine erste Achse (47) drehbar gegen die Aufnahme (2) oder den integrierten Behälter gelagert ist, wobei ein freies Ende (48) des ersten Hebels (46) gegen eine verformbare Seitenwand (50) der Aufnahme (2) oder den integrierten Behälter drückbar ist, wobei das Bedienelement (4) durch einen zweiten Hebel (4) gebildet ist, der um eine zweite Achse (40) drehbar gegen die Aufnahme (2) oder den integrierten Behälter gelagert ist, wobei die zweite Achse (40) den zweiten Hebel (4) in einen kurzen Hebelarm (42) und einen langen Hebelarm (41) teilt, wobei ein Ende des kurzen Hebelarms (42) durch manuelles Bedienen des langen Hebelarms (41) gegen den ersten Hebel (46) zu drücken ist, so dass das freie Ende des ersten Hebels (46) gegen die verformbare Seitenwand (50) drückt und diese derart verformt, dass eine in der Aufnahme (2) befindlicher separater Behälter (54) zu öffnen ist, oder das freie Ende des ersten Hebels (46) gegen den integrierten Behälter drückt, so dass der integrierte Behälter sich zu einer Verbindung zum Pumpraum (28) hin öffnet.

4. Mischvorrichtung nach einem der vorangehenden Ansprüche, **dadurch gekennzeichnet, dass**
das Bedienelement (4) manuell beweglich ist, vorzugsweise ein um eine Achse (40) schwenkbarer Hebel (4) ist, wobei das Bedienelement (4) derart mit der Öffnungseinrichtung (5), der Druckpumpe (3) und der Mischeinrichtung (10) in Wirkverbindung steht oder in Wirkverbindung zu bringen ist, dass bei einem ersten Bedienen des Bedienelements (4) ein separater Behälter (54) in der Aufnahme (2) oder der integrierte Behälter zu öffnen ist und bei einem weiteren Bedienen des Bedienelements (4) der Kolben (30) in der Druckpumpe (3) anzutreiben ist und die Mischeinrichtung (10) im Innenraum anzutreiben ist.

5. Mischvorrichtung nach Anspruch 4, **dadurch gekennzeichnet, dass**
der Kolben (30) der Druckpumpe (3) und/oder die Mischeinrichtung (10) über ein flexibles Kabel (34, 86, 90) und/oder eine Stange anzutreiben sind, wobei an dem flexiblen Kabel (34, 86, 90) und/oder der Stange ein Rastmittel (36) vorgesehen ist, das nach erstmaligem Bedienen des Bedienelements (4) in ein Gegenrastmittel (38) am Bedienelement (4) oder in ein mit dem Bedienelement (4) verbundenes Gegenrastmittel (38) greift, so dass bei einem der Rastung nachfolgenden Bedienen des Bedienelements (4) der Kolben (30) der Druckpumpe (3) und/oder die Mischeinrichtung (10) über das Kabel (34, 86, 90) und/oder die Stange mit dem Bedienelement (4) anzutreiben sind.

6. Mischvorrichtung nach einem der vorangehenden Ansprüche, **dadurch gekennzeichnet, dass**
die Mischeinrichtung (10) durch Bedienen des Bedienelements (4) in dem Innenraum um die Längsachse des Innenraums drehbar ist, wobei hierzu vorzugsweise sich ein mit der Mischeinrichtung (10) verbundener Zylinder (76) mit einem Außengewinde (78) in einer feststehenden Hülse (80) mit einem passenden Innengewinde (82) bewegt, so dass bei einer Bewegung des Zylinders (76) entlang der Längsrichtung innerhalb der Hülse (80) eine Drehung des Zylinders (76) erzwungen wird, wobei sich die Drehung des Zylinders (76) auf die Mischeinrichtung (10) im Innenraum der Kartusche (6) überträgt.

7. Mischvorrichtung nach einem der vorangehenden Ansprüche, **dadurch gekennzeichnet, dass**
der Pumpraum (28) der Druckpumpe (3) flüssigkeitsdicht ist und im Inneren der Druckpumpe (3) angeordnet ist, wobei der Kolben (30) über das Bedienelement (4) manuell in zumindest einer Richtung antreibbar ist, so dass durch die Bewegung des Kolbens (30) der Pumpraum (28) zu verkleinern ist und mit dem sich dadurch verringernden Volumen des Pumpraums (28) der Inhalt des Pumpraums (28), insbesondere die Monomerflüssigkeit im Pumpraum (28), durch die Verbindungsleitung (26) in den Innenraum der zumindest einen Kartusche (6) drückbar ist.

8. Mischvorrichtung nach einem der vorangehenden Ansprüche, **dadurch gekennzeichnet, dass**
der Kolben (30) in einem Hohlzylinder (29) axial beweglich gelagert ist, wobei der Hohlzylinder (29) auf einer dem Kolben (30) gegenüberliegenden ersten Seite geschlossen ist oder bis auf eine Durchführung für eine mit dem Bedienelement (4) und dem Kolben (30) verbundene Stange (34) oder verbundenen Kabel (34) geschlossen ist, insbesondere durch einen Verschluss (33) geschlossen ist, wobei vorzugsweise der Pumpraum (28) in dem Hohlzylinder (29) zwischen dem Kolben (30) und der ersten geschlossenen Seite gebildet ist, wobei besonders bevorzugt der Hohlzylinder (29) auf einer zweiten Seite offen ist.

9. Mischvorrichtung nach Anspruch 8, **dadurch gekennzeichnet, dass**
der Kolben (30) der Druckpumpe (3) über ein flexibles Kabel (34, 90) und/oder eine Stange anzutreiben ist, wobei ein erster Teil des Kabels (34) und/oder die Stange durch den Pumpraum (28) läuft und auf der ersten Seite durch eine flüssigkeitsdichte Durchführung aus dem Pumpraum (28) herausgeführt ist, insbesondere durch eine flüssigkeitsdichte Durchführung durch den Verschluss (33) aus dem Pumpraum (28) herausgeführt ist, wobei der Kolben (30) durch Bedienen des Bedienelements (4) mit dem flexiblen Kabel (34, 90) und/oder der Stange in Richtung in Richtung der ersten Seite des Hohlzylinders (29) zu ziehen ist, so dass sich der Pumpraum (28) verkleinert.

10. Mischvorrichtung nach einem der vorangehenden Ansprüche, **dadurch gekennzeichnet, dass**
die Kartusche (6) eine mit Zementpulver (9) gefüllte Zementkartusche (6) ist und in der Aufnahme (2) ein separater Behälter (54) enthaltend eine Monomerflüssigkeit angeordnet ist oder in dem integrierten Behälter eine Monomerflüssigkeit enthalten ist, wobei vorzugsweise die Aufnahme (2) oder der integrierte Behälter durch ein zu öffnendes Trennelement flüssigkeitsundurchlässig mit dem Pumpraum (28) der Druckpumpe (3) verbunden ist.

11. Mischvorrichtung nach einem der vorangehenden Ansprüche, **dadurch gekennzeichnet, dass**
die Kartusche (6), die Druckpumpe (3) und alle Leitungen (26, 70) sowie die Aufnahme (2) oder der integrierte Behälter mit einem gemeinsamen Fußteil (18) und/oder einem Gehäuse (19) fest und/oder lösbar verbunden sind, wobei vorzugsweise die Druckpumpe (3) und alle Leitungen (26, 70) sowie die Aufnahme (2) oder der separate Behälter (54) fest mit dem Fußteil (18) und/oder einem Gehäuse (19) verbunden sind und die Kartusche (6) lösbar mit dem Fußteil (18) und/oder einem Gehäuse (19) verbunden ist.

12. Mischvorrichtung nach einem der vorangehenden Ansprüche, **dadurch gekennzeichnet, dass**
unterhalb der Aufnahme (2) oder des integrierten Behälters eine Verbindung zur Einleitung der Monomerflüssigkeit in den Pumpraum (28) angeordnet ist.

13. Mischvorrichtung nach einem der vorangehenden Ansprüche, **dadurch gekennzeichnet, dass**
durch die Bewegung des Kolbens (30) in der Druckpumpe (3) im Pumpraum (28) ein Druck auf darin enthaltene Monomerflüssigkeit auszuüben ist, wobei mit dem Druck die Monomerflüssigkeit durch die Verbindungsleitung (26) in den Innenraum der zumindest einen Kartusche (6) zu drücken ist.

14. Mischvorrichtung nach einem der vorangehenden Ansprüche, **dadurch gekennzeichnet, dass**
die Druckpumpe (3) aufgebaut ist mit
einem Hohlzylinder (29), wobei der Hohlzylinder (29) mit dem Innenraum der Kartusche (6) verbunden oder verbindbar ist,
einem flüssigkeitsdichten Verschluss (33) an einem Hohlzylinderende,
dem Kolben (30), der im Hohlzylinder (29) flüssigkeitsdicht, axial beweglich angeordnet ist, wobei
der Kolben (30) in der Druckpumpe (3) mit dem manuell bedienbaren Bedienelement (4) bewegbar ist, wobei
bei einem Bewegen des Kolbens (30) mit dem manuell bedienbaren Bedienelement (4) der Kolben (30) axial in Richtung des Verschlusses (33) bewegbar ist und dadurch eine Monomerflüssigkeit im Pumpraum (28) in den Innenraum der Kartusche (6) drückbar ist, wobei
das Bedienelement (4) mit der Öffnungseinrichtung (5) in Wirkverbindung steht und wobei
das Bedienelement (4) mit der Mischeinrichtung (10) im Innenraum der Kartusche (6) derart verbunden ist, dass bei einem Bedienen des Bedienelements (4) die Mischeinrichtung (10) im Innenraum der Kartusche (6) bewegbar ist.

15. Mischvorrichtung nach einem der vorangehenden Ansprüche, **dadurch gekennzeichnet, dass**
die Druckpumpe (3), die Öffnungseinrichtung (5) und die Mischeinrichtung (10) über die Bewegung des Bedienelements (4) antreibbar sind, wobei bevorzugt die Bewegung des Bedienelements (4) durch manuelle Krafteinwirkung erfolgt.

16. Verfahren zur Vermischung von Polymethylmethacrylat-Knochenzement in einem Innenraum einer Kartusche (6) einer Mischvorrichtung, insbesondere einer Mischvorrichtung nach einem der vorangehenden Ansprüche, wobei ein Bedienelement (4) bedient wird und dadurch ein integrierter Behälter der Mischvorrichtung oder ein separater Behälter (54), der in einer Aufnahme (2) der Mischvorrichtung angeordnet ist, geöffnet wird, wobei anschließend eine in dem integrierten Behälter oder dem separaten Behälter (54) enthaltene Monomerflüssigkeit als erste Komponente des Knochenzements (96) in einen Pumpraum (28) einer Druckpumpe (3) fließt,
durch eine anschließende weitere Bedienung des Bedienelements (4) eine Bewegung eines Kolbens (30) der Druckpumpe (3) der Mischvorrichtung angetrieben wird, wobei durch die Bewegung des Kolbens (30) die Monomerflüssigkeit aus dem Pumpraum (28) der Druckpumpe (3) durch eine Verbindungsleitung (26) in den Innenraum der Kartusche (6) gedrückt wird, wobei sich in dem Innenraum der Kartusche (6) bereits ein Knochenzementpulver (9) als zweite Komponente des Knochenzements (96) befindet, und
durch die Bedienung des Bedienelements (4) eine Mischeinrichtung (10) im Innenraum der Kartusche (6) bewegt wird und durch die Bewegung der Mischeinrichtung (10) ein Knochenzementteig (96) im Innenraum der Kartusche (6) aus dem Zementpulver (9) und der Monomerflüssigkeit gemischt wird.

17. Verfahren nach Anspruch 16, **dadurch gekennzeichnet, dass**
der Kolben (30) der Druckpumpe (3) mit dem Bedienelement (4) bewegt wird, wodurch die in dem Pumpraum (28) enthaltene Monomerflüssigkeit durch eine Verbindungsleitung (26) in den Innenraum der Kartusche (6) gedrückt wird, anschließend durch Bedienen desselben Bedienelements (4) die Mischeinrichtung (10) im Innenraum der Kartusche (6) bewegt wird und dabei das Zementpulver (9) mit der Monomerflüssigkeit vermischt wird, anschließend die Kartusche (6) mit dem gemischten Zementteig (96) entnommen wird und
durch axiale Bewegung eines Austragskolbens (8) der Zementteig (96) aus der Kartusche (6) heraus gepresst wird.

18. Verfahren nach einem der Ansprüche 16 oder 17, **dadurch gekennzeichnet, dass**
das Zementpulver (9) in der Kartusche (6) angeordnet ist,
die Monomerflüssigkeit in einer von der Kartusche (6) separaten Aufnahme (2) angeordnet ist, wobei die Monomerflüssigkeit in einem integrierten Behälter oder in einem separaten Behälter (54), vorzugsweise in einer Glasampulle (54) in der Aufnahme (2), enthalten ist,
der integrierte Behälter oder der separate Behälter (54) durch Bedienen des Bedienelements (4) und einer daraus resultierenden Bewegung der Öffnungseinrichtung (5) geöffnet wird und die Monomerflüssigkeit aus dem Behälter in den Pumpraum (28) fließt, bevor der Kolben (30) durch ein weiteres Bedienen des Bedienelements (4) angetrieben wird, und
danach der Kolben (30) axial in einem Hohlzylinder (29) bewegt wird, wodurch die im Pumpraum (28) befindliche Monomerflüssigkeit durch die Verbindungsleitung (26) in den Innenraum der Kartusche (6) gedrückt wird.

## Claims

1. Mixing device for the mixing of polymethylmethacrylate bone cement from a monomer liquid and a cement powder (9), the mixing device comprising at least one cartridge (6) comprising an internal space, which can be evacuated, for the mixing of the bone cement (96);
a mixing facility (10) for the mixing of the content of the internal space of the at least one cartridge (6) that is arranged such as to be mobile in the internal space;
a receptacle (2) for accommodation of a separate container (54) that contains the monomer liquid or comprising an integrated container containing the monomer liquid;
an opening facility (5) that is arranged in the area of the receptacle (2) such as to be mobile with respect to the receptacle (2) such that a separate container arranged in the receptacle (2) can be opened by the opening facility (5) by means of a motion of the opening facility (5), or the opening facility (5) is arranged in the area of the integrated container such as to be mobile with respect to the integrated container such that the integrated container can be opened by the opening facility (5) by means of a motion of the opening facility (5);
a pressure pump (3), in which a mobile plunger (30) for conveying a liquid is arranged and which limits a pump space (28) of the pressure pump (3); and a connecting line (26) that connects the internal space of the at least one cartridge (6) to the pump space (28) of the pressure pump (3), whereby the mixing device comprises an operating element (4) that can be operated from outside,
whereby the plunger (30) can be moved manually in the pressure pump (3) by means of the operating element (4); and whereby
the opening facility (5) can be moved against the receptacle (2) or against the integrated container by means of the same operating element (4); and
the mixing facility (10) can be moved in the internal space of the cartridge (6) for the mixing of the content of the internal space of the cartridge (6) by means of the same operating element (4).

2. Mixing device according to claim 1, **characterised in that**
the operating element (4) is or can be connected appropriately to the plunger (30) such that the plunger (30) can be moved manually in the pressure pump (3) by operating the operating element (4).

3. Mixing device according to any one of the preceding claims, **characterised in that**
the opening facility (5) comprises a first lever (46) that is supported against the receptacle (2) or the integrated container such that it can rotate about a first axis (47), whereby a free end (48) of the first lever (46) can be pushed against a deformable side wall (50) of the receptacle (2) or the integrated container, whereby the operating element (4) is formed by a second lever (4) that is supported against the receptacle (2) or the integrated container such that it can rotate about a second axis (40), whereby the second axis (40) divides the second lever (4) into a short lever arm (42) and a long lever arm (41), whereby one end of the short lever arm (42) can be pushed against the first lever (46) by manual operation of the long lever arm (41) such that the free end of the first lever (46) pushes against the deformable side wall (50) and deforms said side wall appropriately such that a separate container (54) that is situated in the receptacle (2) can be opened, or the free end of the first lever (46) pushes against the integrated container such that the integrated container opens toward a connection to the pump space (28).

4. Vacuum mixing device according to any one of the preceding claims,
**characterised in that**
the operating element (4) can be moved manually, preferably it is a lever (4) that can be swivelled about an axis (40), whereby the operating element (4) is or can be brought into an operative connection to the opening facility (5), the pressure pump (3), and the mixing facility (10) in appropriate manner such that a separate container (54) in the receptacle (2) or the integrated container can be opened by means of a first operation of the operating element (4), and, by means of another operation of the operating element (4), the plunger (30) can be driven in the pressure pump (3) and the mixing facility (10) can be driven in the internal space.

5. Vacuum mixing device according to claim 4, **characterised in that**
the plunger (30) of the pressure pump (3) and/or the mixing facility (10) can be driven via a flexible cable (34, 86, 90) and/or a rod, whereby the flexible cable (34, 86, 90) and/or the rod have a snap-in means (36) provided on them, which, after the operating element (4) is operated for the first time, engages an opposite snap-in means (38) on the operating element (4) or an opposite snap-in means (38) that is connected to the operating element (4) such that an operation of the operating element (4) after the snapping-in takes place can drive the plunger (30) of the pressure pump (3) and/or the mixing facility (10) via the cable (34, 86, 90) and/or the rod by means of the operating element (4).

6. Mixing device according to any one of the preceding claims, **characterised in that**
the mixing device (10) can be rotated in the internal space about the longitudinal axis of the internal space through operation of the operating element (4), whereby, for this purpose, a cylinder (76) having an external thread (78) that is connected to the mixing facility (10) is preferred to move in a fixed sleeve (80) having a matching internal thread (82) such that the cylinder (76) is forced to perform a rotation upon a motion of the cylinder (76) along the longitudinal direction inside the sleeve (80), whereby the rotation of the cylinder (76) is transferred to the mixing facility (10) in the internal space of the cartridge (6).

7. Mixing device according to any one of the preceding claims, **characterised in that**
the pump space (28) of the pressure pump (3) is liquid-tight and is arranged on the inside of the pressure pump (3), whereby the plunger (30) can be manually driven in at least one direction by means of the operating element (4) such that the motion of the plunger (30) allows the pump space (28) to be decreased and the thus reducing volume of the pump space (28) allows the content of the pump space (28), in particular the monomer liquid in the pump space (28), to be pushed through the connecting line (26) into the internal space of the at least one cartridge (6).

8. Mixing device according to any one of the preceding claims, **characterised in that**
the plunger (30) is supported in a hollow cylinder (29) such as to be axially mobile, whereby the hollow cylinder (29) is closed on a first side that is opposite from the plunger (30) or is closed except for a feed-through for a rod (34) or cable (34) that is connected to the operating element (4) and the plunger (30), in particular is closed by means of a closure (33), whereby the pumping space (28) is preferably formed in the hollow cylinder (29) between the plunger (30) and the first closed side, whereby the hollow cylinder (29) is preferably open on a second side.

9. Mixing device according to claim 8, **characterised in that**
the plunger (30) of the pressure pump (3) can be driven by means of a flexible cable (34, 90) and/or a rod, whereby a first part of the cable (34) and/or the rod extends through the pump space (28) and is guided out of the pump space (28) on the first side through a liquid-tight feed through, in particular is guided out of the pump space (28) through the closure (33) through a liquid-tight feed through, whereby the plunger (30) can be pulled in the direction of the first side of the hollow cylinder (29) by operating the operating element (4) with the flexible cable (34, 90) and/or the rod such that the pump space (28) decreases in size.

10. Mixing device according to any one of the preceding claims, **characterised in that**
the cartridge (6) is a cement powder (9)-filled cement cartridge (6) and a separate container (54) containing a monomer liquid is arranged in the receptacle (2) or a monomer liquid is contained in the integrated container, whereby, preferably, the receptacle (2) or the integrated container is connected in liquid-impermeable manner to the pump space (28) of the pressure pump (3) by means of a separating element that can be opened.

11. Mixing device according to any one of the preceding claims, **characterised in that**
the cartridge (6), the pressure pump (3), and all lines (26, 70) as well as the receptacle (2) or the integrated container are connected, firmly and/or detachably, to a common foot part (18) and/or a casing (19), whereby, preferably, the pressure pump (3) and all lines (26, 70) as well as the receptacle (2) or the separate container (54) are firmly connected to the foot part (18) and/or a casing (19), and the cartridge (6) is detachably connected to the foot part (18) and/or a casing (19).

12. Mixing device according to any one of the preceding claims, **characterised in that**
a connection for feeding the monomer liquid into the pump space (28) is arranged below the receptacle (2) or the integrated container.

13. Mixing device according to any one of the preceding claims, **characterised in that**
the motion of the plunger (30) in the pressure pump (3) allows a pressure to be exerted on the monomer liquid that is contained in the pump space (28), whereby the monomer liquid can be pushed through the connecting line (26) into the internal space of the at least one cartridge (6) by means of said pressure.

14. Mixing device according to any one of the preceding claims, **characterised in that**
the pressure pump (3) is composed of
a hollow cylinder (29), whereby the hollow cylinder (29) is or can be connected to the internal space of the cartridge (6);
a liquid-tight closure (33) on one end of the hollow cylinder;
the plunger (30) that is arranged in liquid-tight manner in the hollow cylinder (29) such as to be axially mobile, whereby
the plunger (30) can be moved in the pressure pump (3) by means of the manually operable operating element (4), whereby
the plunger (30) can be moved axially in the direction of the closure (33) by moving the plunger (30) with the manually operable operating element (4) and, by this means, a monomer liquid in the pump space (28) can be pushed into the internal space of the cartridge (6), whereby
the operating element (4) is in an operative connection with the opening facility (5), and whereby
the operating element (4) is appropriately connected to the mixing facility (10) in the internal space of the cartridge (6) such that the mixing facility (10) can be moved in the internal space of the cartridge (6) by operating the operating element (4).

15. Mixing device according to any one of the preceding claims, **characterised in that**
the pressure pump (3), the opening facility (5), and the mixing facility (10) can be driven by moving the operating element (4), whereby the operating element (4) is preferably moved by the action of a manual force.

16. Method for the mixing of polymethylmethacrylate bone cement in an internal space of a cartridge (6) of a mixing device, in particular of a mixing device according to any one of the preceding claims, whereby
an operating element (4) is being operated and thereby an integrated container of the mixing device or a separate container (54), which is arranged in a receptacle (2) of the mixing device, is being opened, whereby a monomer liquid that is contained in the integrated container or the separate container (54) then flows into a pump space (36) of a pressure pump (3) as first component of the bone cement (96);
a subsequent further operation of the operating element (4) drives a motion of a plunger (30) of the pressure pump (3) of the mixing device, whereby the motion of the plunger (30) pushes the monomer liquid from the pump space (28) of the pressure pump (3) through a connecting line (26) into the internal space of the cartridge (6), whereby a bone cement powder (9) is already present in the internal space of the cartridge (6) as second component of the bone cement (96); and
the operation of the operating element (4) moves a mixing facility (10) in the internal space of the cartridge (6), and the motion of the mixing facility (10) mixes a bone cement dough (96) in the internal space of the cartridge (6) from the cement powder (9) and the monomer liquid.

17. Method according to claim 16, **characterised in that**
the plunger (30) of the pressure pump (3) is moved by means of the operating element (4), by means of which the monomer liquid contained in the pump space (28) is pushed through a connecting line (26) into the internal space of the cartridge (6);
then the mixing facility (10) is moved in the internal space of the cartridge (6) by operation of the same operating element (4) and, in the process, the cement powder (9) is being mixed with the monomer liquid;
then the cartridge (6) containing the mixed cement dough (96) is being removed; and
the cement dough (96) is being pressed from the cartridge (6) by axial motion of a dispensing plunger (8).

18. Method according to any one of the claims 16 or 17, **characterised in that**
the cement powder (9) is arranged in the cartridge (6);
the monomer liquid is arranged in a receptacle (2) that is separate from the cartridge (6), whereby the monomer liquid is contained in an integrated container or in a separate container (54), preferably is contained in a glass ampoule (54) in the receptacle (2);
the integrated container or the separate container (54) is being opened by operation of the operating element (4) and an ensuing motion of the opening facility (5) and the monomer liquid flows from the container into the pump space (28) before the plunger (30) is driven by means of a further operation of the operating element (4); and
then the plunger (30) is moved axially in a hollow cylinder (29), by means of which the monomer liquid that is present in the pump space (28) is being pushed through the connecting line (26) into the internal space of the cartridge (6).

## Revendications

1. Dispositif de mélange destiné à mélanger du ciment osseux à base de polyméthacrylate de méthyle à partir d'un monomère liquide et d'une poudre de ciment (9), le dispositif de mélange comportant
au moins une cartouche (6) comportant un espace intérieur évacuable pour mélanger le ciment osseux (96),
un dispositif de mélange (10) pour mélanger le contenu de l'espace intérieur de l'au moins une cartouche (6), qui est agencée de manière mobile dans l'espace intérieur,
un logement (2) pour la réception d'un récipient séparé (54) contenant le monomère liquide ou comportant un récipient intégré contenant le monomère liquide,
un dispositif d'ouverture (5) agencé dans la zone du logement (2) de manière mobile contre le logement (2), de sorte qu'un mouvement du dispositif d'ouverture (5) permette d'ouvrir un récipient séparé (54) agencé dans le logement (2) à l'aide du dispositif d'ouverture (5), ou que le dispositif d'ouverture (5) soit agencé de manière mobile dans la zone du récipient intégré contre le récipient intégré, de sorte qu'un mouvement du dispositif d'ouverture (5) permette d'ouvrir le récipient intégré à l'aide du dispositif d'ouverture (5),
une pompe de refoulement (3) qui comporte à l'intérieur un piston mobile (30) pour le refoulement d'un liquide, ledit piston délimitant une chambre de pompage (28) de la pompe de refoulement (3), et
une conduite de liaison (26), qui relie l'espace intérieur de l'au moins une cartouche (6) à la chambre de pompage (28) de la pompe de refoulement (3), le dispositif de mélange présentant un élément de commande (4) manoeuvrable de l'extérieur, de sorte
que ledit élément de commande (4) permette de bouger manuellement le piston (30) dans la pompe de refoulement (3), et de sorte qu'il soit possible avec le même élément de commande (4) de mettre en mouvement le dispositif d'ouverture (5) contre le logement (2) ou contre le récipient intégré, et
qu'il soit possible avec le même élément de commande (4) de mettre en mouvement le dispositif de mélange (10) dans l'espace intérieur de la cartouche (6) pour mélanger le contenu de l'espace intérieur de la cartouche (6).

2. Dispositif de mélange selon la revendication 1, **caractérisé en ce, que**
l'élément de commande (4) soit relié ou reliable au piston (30) de telle sorte, que le piston (30) dans la pompe de refoulement (3) puisse être mis en mouvement par l'actionnement de l'élément de commande (4).

3. Dispositif de mélange selon l'une des revendications précédentes, **caractérisé en ce, que**
le dispositif d'ouverture (5) comporte un premier levier (46) tournant autour d'un premier axe (47) contre le logement (2) ou le récipient intégré, une extrémité libre (48) du premier levier (46) pouvant être poussée contre une paroi latérale déformable (50) du logement (2) ou contre le récipient intégré, l'élément de commande (4) étant formé par un deuxième levier (4) tournant autour d'un deuxième axe (40) contre le logement (2) ou le récipient intégré, le deuxième axe (40) divisant le deuxième levier (4) en un bras de levier court (42) et un bras de levier long (41), une extrémité du bras de levier court (42) pouvant être poussée contre le premier levier (46) par l'actionnement manuel du bras de levier long (41), de sorte que l'extrémité libre du premier levier (46) exerce une pression contre la paroi latérale déformable (50) en déformant celle-ci, de sorte à générer l'ouverture d'un récipient séparé (54) logé dans le logement (2), ou que l'extrémité libre du premier levier (46) exerce une pression contre le récipient intégré, de sorte que le récipient intégré s'ouvre sur une liaison à la chambre de pompage (28).

4. Dispositif de mélange selon l'une des revendications précédentes, **caractérisé en ce, que**
l'élément de commande (4) puisse être mis en mouvement manuellement, qu'il soit de préférence un levier (4) pivotant autour d'un axe (40), l'élément de commande (4) étant en liaison fonctionnelle ou pouvant être mis en liaison fonctionnelle avec le dispositif d'ouverture (5), la pompe de refoulement (3) et le dispositif de mélange (10), de sorte qu'un premier actionnement de l'élément de commande (4) permette d'ouvrir un récipient séparé (54) dans le logement (2) ou le récipient intégré et qu'un autre actionnement de l'élément de commande (4) permette d'entraîner le piston (30) dans la pompe de refoulement (3) et d'entraîner le dispositif de mélange (10) dans l'espace intérieur.

5. Dispositif de mélange selon la revendication 4, **caractérisé en ce, que**
le piston (30) de la pompe de refoulement (3) et/ou le dispositif de mélange (10) puissent être actionnés par l'intermédiaire d'un câble flexible (34, 86, 90) et/ou d'une barre, un moyen de crantage (36) étant prévu au câble flexible (34, 86, 90) et/ou à la barre, de sorte que ledit moyen de crantage (36) entre en prise après le premier actionnement de l'élément de commande (4) avec un contre-moyen de crantage (38) à l'élément de commande (4) ou avec un contre-moyen de crantage (38) lié à l'élément de commande (4), de sorte que, suite au crantage, un actionnement de l'élément de commande (4) permette d'entraîner le piston (30) de la pompe de refoulement (3) et/ou le dispositif de mélange (10) par l'intermédiaire du câble (34, 86, 90) et/ou de la barre avec l'élément de commande (4).

6. Dispositif de mélange selon l'une des revendications précédentes, **caractérisé en ce, que**
le dispositif de mélange (10) puisse être tourné dans l'espace intérieur autour de l'axe longitudinal de l'espace intérieur par l'actionnement de l'élément de commande (4), ceci étant de préférence assuré par le mouvement dans une douille fixe (80), ayant un filetage intérieur (82) adéquat, d'un cylindre (76) relié au dispositif de mélange (10) et comportant un filetage extérieur (78), de sorte qu'un mouvement du cylindre (76) le long du sens longitudinal au sein la douille (80) engendre une rotation forcée du cylindre (76), cette rotation du cylindre (76) étant transmise au dispositif de mélange (10) dans l'espace intérieur de la cartouche (6).

7. Dispositif de mélange selon l'une des revendications précédentes, **caractérisé en ce, que**
la chambre de pompage (28) de la pompe de refoulement (3) soit étanche aux liquides et qu'elle soit agencée à l'intérieur de la pompe de refoulement (3), le piston (30) pouvant être entraîné manuellement dans au moins un sens par l'intermédiaire de l'élément de commande (4), de sorte que le mouvement du piston (30) permette de réduire la chambre de pompage (28) et que la réduction du volume ainsi produite de la chambre de pompage (28) permette de presser le contenu de la chambre de pompage (28) - notamment le monomère liquide dans la chambre de pompage (28) - via la conduite de liaison (26) dans l'espace intérieur de l'au moins une cartouche (6).

8. Dispositif de mélange selon l'une des revendications précédentes, **caractérisé en ce, que**
le piston (30) soit monté avec une mobilité axiale dans un cylindre creux (29), le cylindre creux (29) étant fermé sur un premier côté situé en face du piston (30) ou étant fermé jusqu'au passage d'une barre (34) reliée ou d'un câble (34) relié à l'élément de commande (4) et au piston (30), qu'il soit notamment fermé par un élément de fermeture (33), la chambre de pompage (28) dans le cylindre creux (29) étant de préférence formée entre le piston (30) et le premier côté fermé, le cylindre creux (29) étant de préférence particulière ouvert sur un deuxième côté.

9. Dispositif de mélange selon la revendication 8, **caractérisé en ce, que**
le piston (30) de la pompe de refoulement (3) puisse être entraîné par l'intermédiaire d'un câble flexible (34, 90) et/ou d'une barre, de sorte qu'une première partie du câble (34) et/ou la barre chemine au travers de la chambre de pompage (28) et sorte sur le premier côté de la chambre de pompage (28) à travers un passage étanche aux liquides, en particulier par une sortie hors de la chambre de pompage (28) à travers un passage étanche aux liquides au travers de l'élément de fermeture (33), le piston (30) pouvant être tiré dans le sens du premier côté du cylindre creux (29) par l'intermédiaire du câble flexible (34, 90) et/ou de la barre en actionnant l'élément de commande (4), de sorte que la chambre de pompage (28) se réduise.

10. Dispositif de mélange selon l'une des revendications précédentes, **caractérisé en ce, que**
la cartouche (6) soit une cartouche de ciment (6) remplie de poudre de ciment (9) et que soit agencé dans le logement (2) un récipient séparé (54) contenant un monomère liquide ou que le récipient intégré contienne un monomère liquide, le logement (2) ou le récipient intégré étant de préférence relié via un élément de séparation ouvrable de manière imperméable aux liquides avec la chambre de pompage (28) de la pompe de refoulement (3).

11. Dispositif de mélange selon l'une des revendications précédentes, **caractérisé en ce, que**
la cartouche (6), la pompe de refoulement (3) et toutes les conduites (26, 70) ainsi que le logement (2) ou le récipient intégré soient reliés de manière fixe ou démontable avec un socle (18) commun et/ou avec un boitier (19), la pompe de refoulement (3) et toutes les conduites (26, 70) ainsi que le logement (2) ou le récipient séparé (54) étant de préférence reliés de manière fixe avec le socle (18) et/ou avec un boitier (19) et la cartouche (6) de manière démontable avec le socle (18) et/ou avec un boitier (19).

12. Dispositif de mélange selon l'une des revendications précédentes, **caractérisé en ce, que**
soit agencé sous le logement (2) ou sous le récipient intégré un raccordement pour l'introduction du monomère liquide dans la chambre de pompage (28).

13. Dispositif de mélange selon l'une des revendications précédentes, **caractérisé en ce, que**
le mouvement du piston (30) dans la pompe de refoulement (3) génère dans la chambre de pompage (28) une pression sur le monomère liquide qu'elle contient, de sorte qu'avec cette pression, le monomère liquide soit pressé au travers de la conduite de liaison (26) dans l'espace intérieur de l'au moins une cartouche (6).

14. Dispositif de mélange selon l'une des revendications précédentes, **caractérisé en ce, que**
la structure de la pompe de refoulement (3) comporte
un cylindre creux (29), ce cylindre creux (29) étant relié ou pouvant être relié à l'espace intérieur de la cartouche (6),
un élément de fermeture (33) étanche aux liquides à une extrémité du cylindre creux,
le piston (30), qui est agencé de manière étanche aux liquides avec une mobilité axiale dans le cylindre creux (29), de sorte,
que le piston (30) dans la pompe de refoulement (3) soit déplaçable par l'intermédiaire de l'élément de commande (4) pouvant être actionné manuellement, de sorte,
que, lors d'un déplacement du piston (30) à l'aide de l'élément de commande (4) manoeuvrable manuellement, le piston (30) soit déplaçable axialement dans le sens de l'élément de fermeture (33) et qu'il presse ainsi un monomère liquide de la chambre de pompage (28) dans l'espace intérieur de la cartouche (6) et,
que l'élément de commande (4) soit en liaison fonctionnelle avec le dispositif d'ouverture (5) et,
que l'élément de commande (4) soit relié dans l'espace intérieur de la cartouche (6) avec le dispositif de mélange (10) de telle façon, que le dispositif de mélange (10) soit déplaçable dans l'espace intérieur de la cartouche (6) lors de l'actionnement de l'élément de commande (4).

15. Dispositif de mélange selon l'une des revendications précédentes, **caractérisé en ce, que**
la pompe de refoulement (3), le dispositif d'ouverture (5) et le dispositif de mélange (10) puissent être entraînés par le mouvement de l'élément de commande (4), le mouvement de l'élément de commande (4) étant de préférence engendré par un actionnement manuel.

16. Procédé destiné au mélange de ciment osseux à base de polyméthacrylate de méthyle dans un espace intérieur d'une cartouche (6) d'un dispositif de mélange, notamment un dispositif de mélange selon l'une des revendications précédentes, **caractérisé en ce,**
**qu'**un élément de commande (4) soit actionné et que cette action entraîne l'ouverture d'un récipient intégré du dispositif de mélange ou d'un récipient séparé (54) agencé dans un logement (2) du dispositif de mélange, un monomère liquide contenu dans le récipient intégré ou dans le récipient séparé (54) s'écoulant ensuite en tant que premier composant du ciment osseux (96) dans une chambre de pompage (28) d'une pompe de refoulement (3),
**que** par un autre actionnement subséquent de l'élément de commande (4), un piston (30) d'une pompe de refoulement (3) du dispositif de mélange soit entraîné, le mouvement du piston (30) générant une pression sur le monomère liquide de sorte que celui-ci soit poussé hors de la chambre de pompage (28) de la pompe de refoulement (3) au travers d'une conduite de liaison (26) dans l'espace intérieur de la cartouche (6), l'espace intérieur de la cartouche (6) contenant déjà une poudre de ciment osseux (9) en tant que deuxième composant du ciment osseux (96), et
**que** l'actionnement de l'élément de commande (4) génère un mouvement d'un dispositif de mélange (10) dans l'espace intérieur de la cartouche (6) et que ce mouvement du dispositif de mélange (10) génère le mélange d'une pâte de ciment osseux (96) dans l'espace intérieur de la cartouche (6) à partir de la poudre de ciment (9) et du monomère liquide.

17. Procédé selon la revendication 16, **caractérisé en ce, que**
que le piston (30) de la pompe de refoulement (3) soit mis en mouvement par l'intermédiaire de l'élément de commande (4), ce qui a pour effet de pousser le monomère liquide contenu dans la chambre de pompage (28) au travers d'une conduite de liaison (26) dans l'espace intérieur de la cartouche (6),
qu'ensuite, le dispositif de mélange (10) soit mis en mouvement dans l'espace intérieur de la cartouche (6) par l'actionnement du même élément de commande (4), ce qui entraîne le mélange de la poudre de ciment (9) avec le monomère liquide,
qu'ensuite, la cartouche (6) avec la pâte de ciment (96) soit retirée et que la pâte de ciment (96) soit pressée hors de la cartouche (6) par le mouvement axial d'un piston d'extraction (8).

18. Procédé selon l'une des revendications 16 ou 17, **caractérisé en ce, que**
la poudre de ciment (9) soit agencée dans la cartouche (6),
le monomère liquide soit agencé dans un logement (2) séparé de la cartouche (6), le monomère liquide étant contenu dans un récipient intégré ou dans un récipient séparé (54), de préférence dans une ampoule en verre (54) dans le logement (2),
le récipient intégré ou le récipient séparé (54) soit ouvert par l'actionnement de l'élément de commande (4) et par le mouvement du dispositif d'ouverture (5) qui en résulte et que le monomère liquide s'écoule du récipient dans la chambre de pompage (28), avant que le piston (30) soit entraîné par un autre actionnement de l'élément de commande (4), et
qu'ensuite le piston (30) soit mis en mouvement dans un cylindre creux (29), ce qui a pour effet de pousser le monomère liquide contenu dans la chambre de pompage (28) au travers d'une conduite de liaison (26) dans l'espace intérieur de la cartouche (6).
